# EUROPEAN PATENT SPECIFICATION

(11) **EP 4 082 325 B1**
(45) Date of publication and mention of the grant of the patent: **17.04.2024**
(21) Application number: 20906791.7
(22) Date of filing: 04.12.2020
(51) Int. Cl.: A01D 46/24, A01G 3/02, A01D 46/30

(54) **CROP HARVESTING SYSTEM**
ERNTEVORRICHTUNG
SYSTÈME DE RÉCOLTE DE CULTURE

(30) Priority: 25.12.2019 JP 2019234339
(43) Date of publication of application: 02.11.2022
(73) Proprietor: DENSO CORPORATION, Kariya-city, Aichi 448-8661 (JP)
(72) Inventor: INADA, Seisyo, Kariya- city, Aichi 4488661 (JP); NISHINO, Hideyuki, Kariya- city, Aichi 4488661 (JP); ARIHARA, Hiromu, Kariya- city, Aichi 4488661 (JP); HANDA, Gou, Kariya- city, Aichi 4488661 (JP); HASEGAWA, Takaomi, Kariya- city, Aichi 4488661 (JP)
(74) Representative: Kuhnen & Wacker Patent- und Rechtsanwaltsbüro PartG mbB
(86) International application number: PCT/JP2020/045279
(87) International publication number: WO 2021/131619

(56) References cited:
- WO-A1-2016/123656
- WO-A1-2017/152224
- WO-A1-2018/203337
- CN-U- 204 157 295
- JP-A- H04 293 433
- JP-A- H08 194 534
- JP-A- 2000 083 575
- JP-A- 2014 097 038
- JP-A- 2014 183 841
- JP-B1- 5 744 357
- JP-B2- 2 802 638
- JP-Y2- 2 512 024
- JP-Y2- H0 432 043
- JP-Y2- H0 432 043

## Description

### [Technical Field]

The present disclosure relates to a crop harvesting system.

### [Background Art]

In recent years, harvesting apparatuses for automatically harvesting crops have been suggested. For example, harvesting apparatuses for harvesting fruits or the like are considered to be equipped with a harvesting tool having scissors-shaped blades, for example, at an end of a robot arm. Such a harvesting apparatus is mounted on a moving apparatus that is can automatically drive in farm such as a rice field, a vegetable field, a fruit garden, or the like, and is moved to an individual object that is a harvesting object. Thus, the harvesting apparatus can separate the harvesting object from a main stem or branch and harvest by cutting a stalk or a branch with the scissors-shaped harvesting tool.

The harvesting tool herein is brought into direct contact with plants that are harvesting objects. In a case in which one of the individual objects cultivated in the farm is affected with a disease, harvesting other individual objects with the harvesting tool having been in contact with the individual object affected with the disease can spread the disease all over the farm. In this case, workers can manually disinfect the harvesting apparatus; but disinfecting operations will cost a lot of effort, which lowers the operating efficiency in harvesting operations.

### [Citation List]

### [Patent Literature]

[Patent Literature 1] Japanese Unexamined Patent Application Publication No. 2000-092952. JP H0432043 and WO 2016/123656 provide further examples of automatic crop harvesting systems equipped with a disinfection apparatus.

### [Summary of the Invention]

It is an object of the present disclosure to provide a crop harvesting system in which a harvesting apparatus that can automatically harvest harvesting objects is further able to automatically disinfect at least a part to be brought into direct contact with the harvesting objects, such as a harvesting tool or the like. The technical problem is solved by means of a crop harvesting system having the features of claim 1.

According to one aspect of the present disclosure, a crop harvesting system includes a moving apparatus configured to be movable in a harvesting operation area that is an area in which a harvesting operation takes place; a harvesting apparatus mounted on the moving apparatus and harvest a harvesting object by making contact with a crop, the crop being a harvesting object; a disinfecting apparatus having a function of disinfecting at least a disinfection target part of the harvesting apparatus, which is being brought into contact with the harvesting object; a disinfection necessity judgment processing portion to perform a disinfection necessity judgment process of judging whether disinfection to the disinfection target part is necessary; a disinfecting operation processing portion to, when it is judged in the disinfection necessity judgment process that the disinfection is necessary, perform a disinfecting operation of disinfecting the disinfection target part by driving at least one of the harvesting apparatus and the disinfecting apparatus, a location information obtaining apparatus configured to obtain location information of a present location of the moving apparatus; and a present location estimating processing apparatus configured to perform a present location estimating process of estimating the present location of the moving apparatus with reference to the location information obtained by the location information obtaining apparatus. The disinfection necessity judgment process includes a process of judging that the disinfecting operation by the disinfecting apparatus is necessary when it is judged that the harvesting apparatus is going to start the harvesting operation, with reference to the present location of the moving apparatus, the present location being estimated in the present location estimating process, and the disinfecting operation processing portion performs the disinfecting operation before the harvesting apparatus performs the harvesting operation.

Accordingly, at least a disinfection target part to be brought into direct contact with harvesting objects will be able to be automatically disinfected. Since workers do not need to manually disinfect the harvesting apparatus, disinfecting operations will cost less effort than in a case in which they manually disinfect the harvesting apparatus, which results in an increase in the operating efficiency in harvesting operations. Furthermore, the workers will not accidentally forget disinfect it as in the case in which they manually disinfect the harvesting apparatus. Consequently, even in a case in which one individual object in the farm is affected with a disease, the spread of the disease in the farm by the medium of the harvesting tool will be able to be reduced to a minimum.

### [Brief Description of the Drawings]

The above-described object and other objects, characteristics, and advantages of the present disclosure will be more clarified with reference to the attached drawings by a detailed description provided below.
Fig. 1 is a conceptual diagram illustrating an example of the configuration of a crop harvesting system according to a first embodiment;
Fig. 2 is an enlarged view of a part X2 in Fig. 1 in relation to the crop harvesting system according to the first embodiment;
Fig. 3 is a conceptual diagram illustrating an example of the configuration of a disinfecting apparatus in relation to the crop harvesting system according to the first embodiment;
Fig. 4 is a conceptual plan view illustrating an example of the configurations of a spraying portion and a spray sensor of the disinfecting apparatus in relation to the crop harvesting system according to the first embodiment;
Fig. 5 is a block diagram illustrating an example of the electrical configuration of the crop harvesting system according to the first embodiment;
Fig. 6 is a conceptual plan view illustrating an example of the configuration of farm in which the crop harvesting system according to the first embodiment is applied;
Fig. 7 is a flowchart illustrating an example of the disinfection-related process to be invoked by a control apparatus in relation to the crop harvesting system according to the first embodiment;
Fig. 8 is a flowchart illustrating an example of a disinfection necessity judgment process in relation to the crop harvesting system according to the first embodiment;
Fig. 9 is a flowchart illustrating an example of a disinfection operating process in relation to the crop harvesting system according to the first embodiment;
Fig. 10 is a flowchart illustrating an example of the disinfection necessity judgment process in relation to the crop harvesting system according to a second embodiment;
Fig. 11 is a flowchart illustrating an example of the disinfection necessity judgment process in relation to the crop harvesting system according to a third embodiment;
Fig. 12 is a flowchart illustrating an example of the disinfection necessity judgment process in relation to the crop harvesting system according to a fourth embodiment;
Fig. 13 is a flowchart illustrating an example of the disinfection necessity judgment process in relation to the crop harvesting system according to a fifth embodiment;
Fig. 14 is a flowchart illustrating an example of the disinfection necessity judgment process in relation to the crop harvesting system according to a sixth embodiment;
Fig. 15 is a conceptual plan view illustrating an example of the configuration of farm in which the crop harvesting system according to a seventh embodiment is applied; and
Fig. 16 is a flowchart illustrating an example of the disinfection-related process to be invoked by the control apparatus in relation to the crop harvesting system according to the seventh embodiment.

### [Description of the Embodiments according to the invention]

Hereinafter, a plurality of embodiments will be described with reference to the drawings. To avoid repetition in the description, substantially identical configurations are given the same reference numerals in these embodiments.

### (First Embodiment)

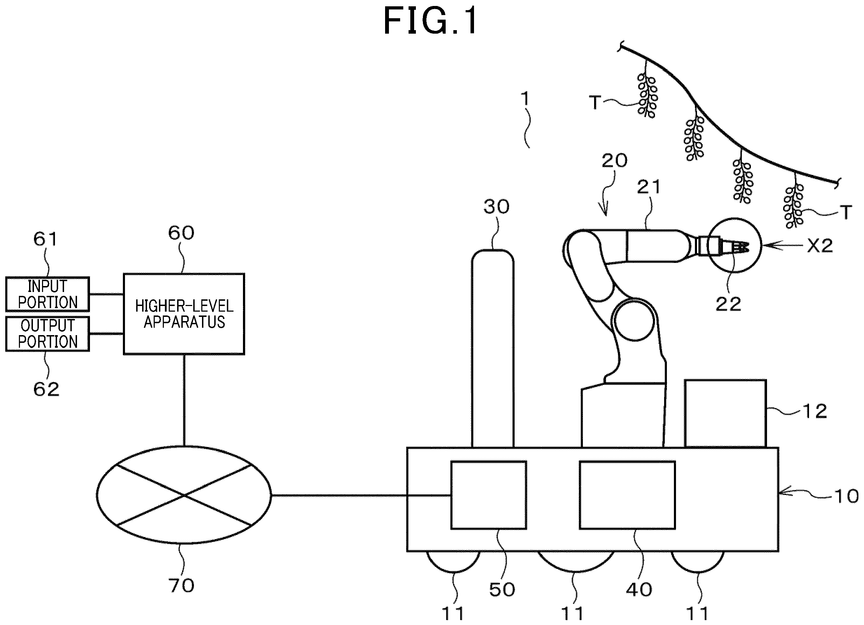

Hereinafter, a first embodiment will be described with reference to Figs. 1 to 9.

### [Configuration of Farmland]

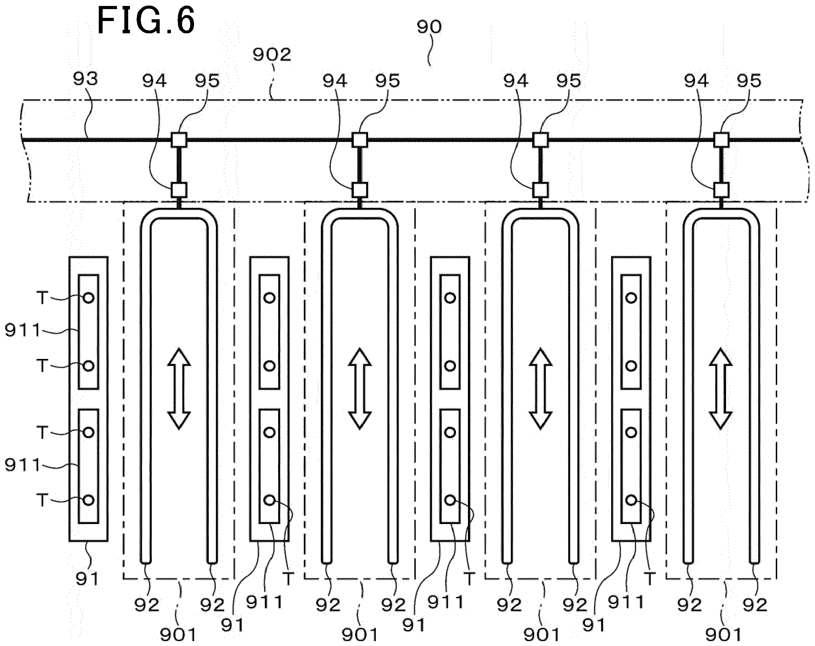

Fig. 1 illustrates a crop harvesting system 1 that is a system for harvesting crops T cultivated in farm such as a rice field, a vegetable field, a fruit garden, a vinyl greenhouse, or what is called a plant factory. In the present embodiment, the farm is intended to represent a vinyl greenhouse or a plant factory, for example, as illustrated in Fig. 6.

The crop harvesting system 1 is used in farm 90, for example, with a farm layout as illustrated in Fig. 6. For example, the farm 90 is provided with a plurality of lanes 91, a plurality of rails 92, and a plurality of trace lines 93. Each of the lanes 91 has a plurality of cultivating devices 911. The cultivating device 911 serves to cultivate the crops T which are harvesting objects. One the lane 91 is composed of a lot of the cultivating devices 911 arranged in line. For example, one the lane 91 measures one or more dozen meters, extending in one direction. In the farm 90, the plurality of the lanes 91 are evenly and adjacently spaced such that they are in parallel.

The rails 92 are provided between two adjoining the lanes 91, extending along the lanes 91. The rails 92 constitute a pair of the rails 92. For example, the trace lines 93 are installed by being attached, printed, or drawn on the land of the farm 90 and connect the rails 92. In this case, an area in which the rails 92 are provided is referred to as a harvesting operation area 901. The area outside the harvesting operation areas 901 and connecting the harvesting operation areas 901, in which the trace lines 93 are provided in this case, is referred to as a connecting area 902.

Furthermore, the farm 90 can be provided with a plurality of signs 94 and 95. The signs 94 and 95 indicate a boundary between the harvesting operation area 901 and the connecting area 902 or a branch point on the trace line 93. In the case of the present embodiment, the sign 94 is provided near the boundary between the harvesting operation area 901 and the connecting area 902; specifically, it is provided at a location in the connecting area 902 but immediately adjacent to the harvesting operation area 901. The sign 95 is provided at a branch point at which a branch diverges from the trace line 93 which is provided such that it traverses by the harvesting operation areas 901. The signs 94 and 95 can be composed of an information transmitting medium that is can transmit information in a contactless manner, using magnetism, radio waves, images, sound waves, or the like. Specifically, the signs 94 and 95 can be composed of, for example, an RFID, a two-dimensional code, a magnetic marker, or the like.

### [Configuration of the Farm Products Harvesting System]

Hereinafter, the crop harvesting system 1 will be described. The crop harvesting system 1 is provided with a moving apparatus 10, a harvesting apparatus 20, a location information obtaining apparatus 30, and a control apparatus 50, as illustrated in Fig. 1. The moving apparatus 10 is equipped with the harvesting apparatus 20, the location information obtaining apparatus 30, a disinfecting apparatus 40, and the control apparatus 50. For example, the moving apparatus 10 has wheels 11 and motors for driving these wheels 11, not shown in the figure. The moving apparatus 10 can drive in the farm 90 in accordance with instructions from a higher-level apparatus 60 that is connected with the control apparatus 50 in a communicable manner, or autonomously in accordance with instructions from the control apparatus 50 mounted on the moving apparatus 10. The moving apparatus 10 can thus move the harvesting apparatus 20, the location information obtaining apparatus 30, the disinfecting apparatus 40, and the control apparatus 50, which are mounted on the moving apparatus 10, to a target location for the harvesting objects T or the like in the farm 90.

In the case of the present embodiment, the wheel 11 has: a tire part to be in contact with a ground and the land of the farm 90; and a tread part to be in contact with the rails 92, details of which are not shown in the figure. The moving apparatus 10 is thus configured to be able to drive on the ground, the land of the farm 90, and the rail 92. In a case in which the moving apparatus 10 drives on the ground or the land of the farm 90, the moving apparatus 10 will be able to recognize the trace line 93 with a camera or the like mounted on the moving apparatus 10 and move along the trace line 93. The moving apparatus 10 further will be able to move along the rail 92 while the tread parts of the wheels 11 are physically guided by the rail 92.

The moving apparatus 10 may be equipped with a collecting box 12. The collecting box 12 is a box for receiving and collecting the harvesting objects T harvested by the harvesting apparatus 20. For example, the collecting box 12 has a shape of a container whose top is open, and is mounted on the moving apparatus 10. For example, in a case in which an apparatus to convey the harvesting objects T, such as a belt conveyor or the like, is installed in the farm 90, the collecting box 12 can be omitted. The collecting box 12 may be configured to follow the moving apparatus 10 by a moving apparatus other than the moving apparatus 10.

The harvesting apparatus 20 is mounted on the moving apparatus 10 and configured to be movable in a unified manner with the moving apparatus 10. The harvesting apparatus 20 has a function of harvesting the crops T, which are harvesting objects, in contact with the harvesting objects T. For example, the harvesting apparatus 20 can be configured to have: an articulated robot arm 21; and a harvesting tool 22 attached to an arm end part of the robot arm 21, as illustrated in Fig. 1.

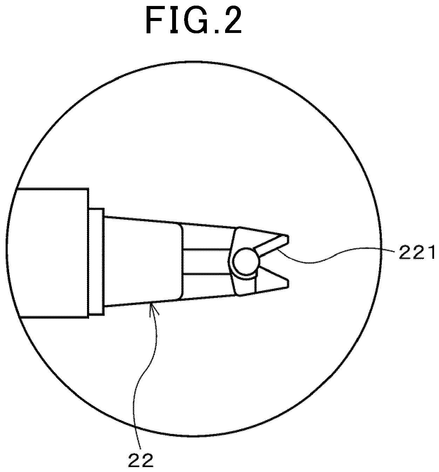

The robot arm 21 can change the position and posture of the harvesting tool 22 freely within the range of motion of the robot arm 21. For example, the harvesting tool 22 is configured to have a shape of scissors having a blade part 221, as illustrated in Fig. 2, and can harvest the harvesting object T by cutting a stalk or branch of the harvesting object T with the blade part 221 in direct contact with the stalk or branch. The harvesting tool 22 should not be limited to a shape of scissors; it may have saw-like blades, for example, or another shape.

The location information obtaining apparatus 30 has a function of obtaining information around the moving apparatus 10 as location information of the present location of the moving apparatus 10, using at least one of magnetism, radio waves, images, and videos. The method of obtaining location information of the present location of the moving apparatus 10 using, for example, magnetism, radio waves, or images is an RFID, a two-dimensional code, a magnetic marker, a visual apparatus, a GPS, or the like. RFID is short for radio frequency identifier. GPS is short for Global Positioning System. The location information obtaining apparatus 30 can obtain location information of the present location of the moving apparatus 10 using a GPS.

The method of obtaining location information of the moving apparatus 10 using a magnetic marker using magnetism, an RFID using radio waves, or a two-dimensional code using images is considered to be applied in the following configuration, for example. That is, a magnetic marker, an RFID, or a two-dimensional code serves as a recording medium that is capable of recording location information, and is installed on a route in the farm 90 along which the moving apparatus 10 moves. The magnetic marker, the RFID, or the two-dimensional code records location information of the installation location of itself and serves as the signs 94 and 95 on the route along which the moving apparatus 10 moves. The location information obtaining apparatus 30 can obtain location information of the present location of the moving apparatus 10 in the farm 90 by reading, respectively, the magnetic marker, the RFID, or the two-dimensional code installed on the route, using magnetism, radio waves, or a camera.

The method of obtaining location information of the present location of the moving apparatus 10 using images or videos is the following method, for example. That is, the control apparatus 50 records in advance images or videos of a view surrounding the route along which the moving apparatus 10 moves. The location information obtaining apparatus 30 obtains images or videos of the view surrounding the moving apparatus 10 as the location information of the present location of the moving apparatus 10. In this case, the control apparatus 50 compares the images or videos obtained by the location information obtaining apparatus 30 to the prerecorded images or videos of the view while the moving apparatus 10 is moving. The control apparatus 50 can thus estimate the present location of the moving apparatus 10 at a point with the best match rate.

The location information obtaining apparatus 30 may not necessarily be configured to be mounted on the moving apparatus 10 and move along with the moving apparatus 10. For example, the location information obtaining apparatus 30 may be composed of one or more cameras or the like attached to a structure in the farm 90. In a case in which the location information obtaining apparatus 30 is composed of a camera or the like attached to a structure in the farm 90, the location information obtaining apparatus 30 will be able to obtain images or videos of the moving apparatus 10 as the location information of the moving apparatus 10 while the moving apparatus 10 is moving in the farm 90, and to estimate the location of the moving apparatus 10 with reference to the images or videos.

The disinfecting apparatus 40 has a function of disinfecting at least a disinfection target part of the harvesting apparatus 20, which is to be brought into contact with the harvesting objects T. In the case of the present embodiment, the harvesting apparatus 20 harvests the harvesting object T by cutting, for example, a stalk or branch of the harvesting target T with the scissors-shaped harvesting tool 22 which is attached to the arm end part of the robot arm 21. Thus, in the case of the present embodiment, a part of the harvesting apparatus 20, which is to be brought into direct contact with the harvesting object T, is the blade part 221 of the harvesting tool 22 illustrated in Fig. 2. In the present embodiment, the disinfection target part includes at least the blade part 221 of the harvesting tool 22. Thus, in the case of the present embodiment, the blade part 221 is also referred to as the disinfection target part 221.

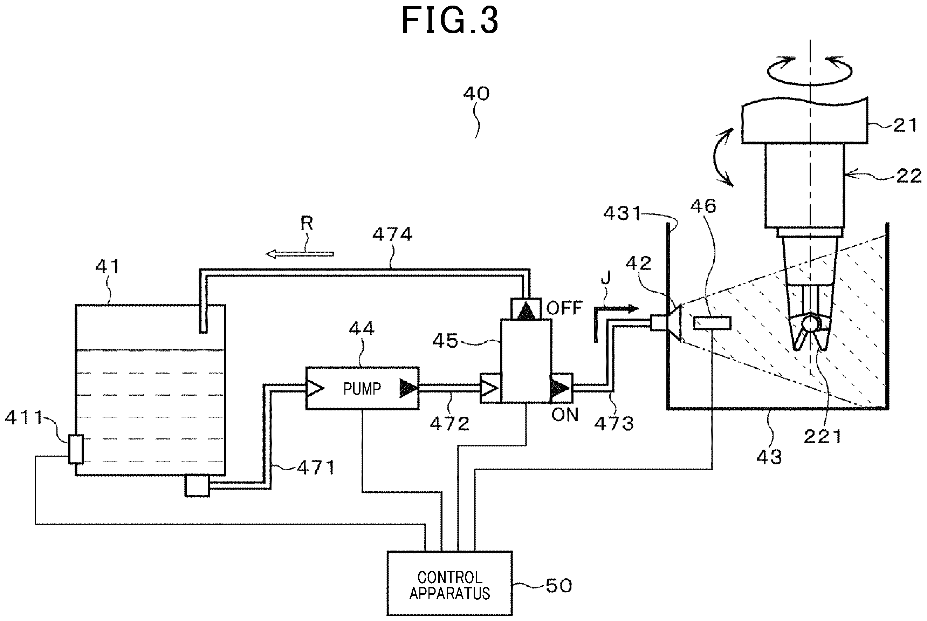

For example, as illustrated in Fig. 3, the disinfecting apparatus 40 has a function of disinfecting a part adjacent to the blade part 221 of the harvesting tool 22, including the blade part 221, which is a disinfection target part, by spraying atomized disinfectant solution i.e., spraying disinfectant solution to the part including the blade part 221. In the present embodiment, ethanol with a concentration of approximately 70% can be used as the disinfectant solution. In this case, the disinfectant solution can be, for example, an alcohol formulation for disinfection a basis of which is ethyl alcohol. This disinfectant solution can be used for sanitary control of food contact tools and materials such as tableware and for frontal disinfection of foods, and is less corrosive to metal than other alcohol formulations.

The disinfecting apparatus 40 is mounted on the moving apparatus 10. As illustrated in Fig. 3, the disinfecting apparatus 40 has a disinfectant solution tank 41, a spraying portion 42, a covering member 43, a pump 44, a three-way solenoid valve 45, and a spraying sensor 46. The disinfectant solution tank 41 and the pump 44 are connected by a piping 471; the pump 44 and the three-way solenoid valve 45 are connected by a piping 472; the three-way solenoid valve 45 and the spraying portion 42 are connected by a piping 473; and the three-way solenoid valve 45 and the disinfectant solution tank 41 are connected by a piping 474.

The disinfectant solution tank 41 is a tank for storing the disinfectant solution. For example, the disinfectant solution tank 41 is composed of, for example, a light-shielding and chemical-resistant member and configured to be able to be covered tightly with, for example, a lid or the like. The disinfectant solution tank 41 is further configured to be able to store a sufficient quantity of the disinfectant solution enough to disinfect the harvesting tool 22 multiple times. The disinfectant solution tank 41 is mounted at a position of the moving apparatus 10, which allows external supply of the disinfectant solution.

For example, workers can manually supply the disinfectant solution tank 41 with the disinfectant solution. Alternatively, for example, a storage tank storing a large quantity of the disinfectant solution may be installed in the farm 90, and the disinfecting apparatus 40 may be configured to be moved to the storage tank by the moving apparatus 10 and automatically supply the disinfectant solution tank 41 from the storage tank.

Furthermore, the disinfectant solution tank 41 has a solution quantity sensor 411. The solution quantity sensor 411 is attached to the disinfectant solution tank 41 at a predetermined height position. For example, the disinfectant solution tank 41 is configured to have electrodes or the like and may detect the level of the surface of the disinfectant solution in the disinfectant solution tank 41 with reference to a change or the like in the resistance or capacitance of the electrodes. The solution quantity sensor 411 is connected with the control apparatus 50. In a case in which the surface of the disinfectant solution in the disinfectant solution tank 41 goes below the installation location of the solution quantity sensor 411, i.e., in a case in which the quantity of the remaining disinfectant solution in the disinfectant solution tank 41 becomes less than a predetermined quantity, the solution quantity sensor 411 outputs a detection signal that notifies the quantity of the remaining disinfectant solution performs short, to the control apparatus 50.

The spraying portion 42 is a nozzle, for example, having a function of extensively spraying an atomized disinfectant solution; and the spraying portion 42 is provided such that it faces the inside of the covering member 43. In the case of the present embodiment, the disinfecting apparatus 40 has one the spraying portion 42. Alternatively, the disinfecting apparatus 40 may have a plurality of the spraying portions 42. In a case in which the disinfecting apparatus 40 has a plurality of the spraying portions 42, it is preferred that these spraying portions 42 be configured to eject a cleansing liquid at different angles.

If droplets of the disinfectant solution sprayed from the spraying portion 42 adhere to the harvesting apparatus 20, another apparatus, a peripheral apparatus that is not a disinfection target, a plant that is not a disinfection target, or a harvesting object having been harvested, they can adversely affect operations of the apparatus, cultivation of the plant, the quality of the harvesting object, or the like. Furthermore, the crop harvesting system 1 according to the present embodiment is assumed to be used outdoors as well. In this case, if the disinfectant solution sprayed from the spraying portion 42 is blown by the wind, the disinfectant solution would not hit the harvesting tool 22, a disinfection target, which results in an impossibility of ensuring that the harvesting tool 22 is disinfected.

As a solution, the disinfecting apparatus 40 has the covering member 43. The spraying portion 42 is enclosed by the covering member 43. The covering member 43 serves to prevent the disinfectant solution, which is sprayed from the spraying portion 42, from scattering in all directions. The covering member 43 also serves to prevent the disinfectant solution, which is sprayed from the spraying portion 42, from being carried away by the wind.

In this case, the covering member 43 has at least one lateral face that faces the spraying portion 42. So, the covering member 43 prevents the disinfectant solution, which is sprayed from the spraying portion 42, from flying out of the covering member 43, by catching the disinfectant solution. In the case of the present embodiment, the covering member 43 is provided on the moving apparatus 10, within the range of motion of the robot arm 21. Furthermore, the covering member 43 is metallic or resinous and configured to have a shape of a container having at least one open part. In the case of the present embodiment, the covering member 43 has an opening portion 431 on its top face. To be disinfected, the harvesting tool 22 is inserted in the covering member 43 through the opening portion 431 of the covering member 43. Then, the spraying portion 42 ejects the disinfectant solution toward the harvesting tool 22 which is inside the covering member 43.

The pump 44, the three-way solenoid valve 45, and the spraying sensor 46 are electrically connected with the control apparatus 50 and driven under the control of the control apparatus 50. The pump 44 is provided between the disinfectant solution tank 41 and the three-way solenoid valve 45. The pump 44 sucks the disinfectant solution stored in the disinfectant solution tank 41 and ejects it toward the three-way solenoid valve 45.

The three-way solenoid valve 45 is, for example, a solenoid valve for liquid. The three-way solenoid valve 45 has a function of selectively switching, in accordance with instructions from the control apparatus 50, between the route indicated by a solid arrow in Fig. 3, i.e., the route through which the disinfectant solution sprayed from the pump 44 is fed to the spraying portion 42 and the route indicated by an empty arrow in Fig. 3, i.e., the route through which the disinfectant solution sprayed from the pump 44 is returned to the disinfectant solution tank 41.

In the following description, the route indicated by the solid arrow in Fig. 3, i.e., the route through which the disinfectant solution sprayed from the pump 44 is fed to the spraying portion 42 is also referred to as a spraying route J. The route indicated by the empty arrow in Fig. 3, i.e., the route through which the disinfectant solution fed from the pump 44 is fed to the spraying portion 42 is also referred to as a return route R. In other words, in the present embodiment, the spraying route J is a route starting from the disinfectant solution tank 41 and reaching the spraying portion 42, by way of the pump 44 and the three-way solenoid valve 45, as indicated by the solid arrow in Fig. 3. The return route R is a route starting from the disinfectant solution tank 41 and returning to the spraying portion 42, by way of the pump 44 and the three-way solenoid valve 45, as indicated by the empty arrow in Fig. 3.

In the OFF state i.e., an initial state in which a solenoid is not driven with no driving voltage applied, the three-way solenoid valve 45 closes the spraying route J between the pump 44 and the spraying portion 42 and opens the return route R between the pump 44 and the disinfectant solution tank 41. When the pump 44 is driven in this state, the disinfectant solution in the disinfectant solution tank 41 is sucked by the pump 44, conveyed by way of the three-way solenoid valve 45, and returned to the disinfectant solution tank 41. The disinfectant solution is circulated by way of the disinfectant solution tank 41, the pump 44, and the three-way solenoid valve 45. Thus, the pipings 471 and 472 of the spraying route J composed of the pipings 471, 472, and 473 and leading from the disinfectant solution tank 41 to the spraying portion 42, which connect, respectively, the disinfectant solution tank 41 to the pump 44 and the pump 44 to the three-way solenoid valve 45, become filled with the disinfectant solution. This causes an increase in the pressure in the piping 472 on a spraying side of the pump 44.

In the ON state in which a solenoid is driven with driving voltage applied, the three-way solenoid valve 45 closes the return route R between the pump 44 and the disinfectant solution tank 41 and opens the spraying route J between the pump 44 and the spraying portion 42. The disinfectant solution sprayed from the pump 44 is thereby fed to the spraying portion 42 and sprayed from the spraying portion 42.

In fact, there is a need for a control of the spraying of the disinfectant solution as much precise as possible, shortening of the duration of spraying, and minimization of the quantity of the consumed disinfectant solution. In the present embodiment, the duration of spraying is thus intended to roughly represent, for example, a few tenths of a second to two seconds. In this case, if the spraying route J is configured to connect the pump 44 directly to the spraying portion 42, i.e., if the spraying route J is configured to omit the return route R including the three-way solenoid valve 45, the disinfectant solution would be sprayed from the spraying portion 42 after any trapped air is expelled from the pipings of the spraying route J. For this reason, there would be a difference in time between the start of driving the pump 44 and the start of spraying the disinfectant solution by the spraying portion 42. In this case, the duration of spraying the disinfectant solution needs to be long enough to properly disinfect the blade part 221 of the harvesting tool 22, which results in an increase in the quantity of the consumed disinfectant solution.

As a solution, in the present embodiment, before spraying the disinfectant solution from the spraying portion 42, the disinfecting apparatus 40 drives the pump 44 for a predetermined period such as roughly 10 seconds to 1 minute while the return route R is open. The disinfecting apparatus 40 thereby circulates the disinfectant solution by way of the disinfectant solution tank 41, the pump 44, and the three-way solenoid valve 45. Thus, any trapped air is expelled from at least the pipings 471 and 472 of the spraying route J composed of the pipings 471, 472, and 473 and leading from the disinfectant solution tank 41 to the three-way solenoid valve 45, and these pipings become filled with the disinfectant solution. This causes an increase in the pressure in the piping 472. The three-way solenoid valve 45 is then switched to the ON state to open the spraying route J. Upon the three-way solenoid valve 45 being switched to the ON state, the disinfectant solution is instantly sprayed from the spraying portion 42 with little possibility of air to be sprayed therefrom. This enables a precise control of the spraying of the disinfectant solution, shortening of the duration of spraying, and minimization of the quantity of the consumed disinfectant solution.

The spraying sensor 46 serves to detect whether the disinfectant solution is actually being sprayed from the spraying portion 42. The spraying sensor 46, which serves to detect whether the disinfectant solution is being sprayed from the spraying portion 42, is intended to represent a pressure sensor, a flow sensor, a rain sensor, or a condensation sensor. In a case in which a small quantity of the disinfectant solution is sprayed for a short time as in the present embodiment, however, a pressure sensor, a flow sensor, a rain sensor, and a condensation sensor are considered inadequate for the following reasons.

That is, in a case in which a pressure sensor is used to detect spraying of the disinfectant solution, it is considered that the pressure sensor detects the pressure in the piping close to the spraying portion 42 and estimates the state of spraying of the spraying portion 42 with reference to a change in the pressure. However, a method of estimating spraying of the disinfectant solution with a pressure sensor would require a long measurement time of a certain degree and is considered not adequate for a short-time spraying. Therefore, a pressure sensor is considered inadequate in a case in which a relatively short-time detection is required as in the present embodiment.

In another case in which a flow sensor is used to detect spraying of the disinfectant solution, it is considered that the flow sensor detects the flow rate of the disinfectant solution in the piping close to the spraying portion 42 and estimates the state of spraying of the spraying portion 42 with reference to a change in the flow rate. However, flow sensors are generally expensive. Particularly, flow sensors that can detect a very small quantity are even more expensive. Therefore, in a case in which a relatively small quantity of the disinfectant solution is sprayed at a time as in the present embodiment, a flow sensor is considered inadequate for the detection of the disinfectant solution.

Rain sensors or condensation sensors detect whether they are soaked, with reference to a resistance that changes by the surfaces getting soaked. In a case in which such a rain sensor or condensation sensor is used, the rain sensor or the condensation sensor should be mounted at a position at which it will be subjected to the adhesion of the disinfectant solution sprayed from the spraying portion 42. The rain sensor or the condensation sensor can thus estimate spraying of the disinfectant solution by detecting a change in the resistance of the sensor. However, rain sensors or condensation sensors require a certain time until they are soaked enough to detect a change in the resistance. Furthermore, once getting soaked and detecting the disinfectant solution on the surfaces, rain sensors or condensation sensors must wait until the surfaces are dried out and ready to be used again. Furthermore, rain sensors or condensation sensors change the resistance also by being subjected to the adhesion of other liquids such as condensation. Therefore, in a particular case in which they are used in a high temperature and humidity environment such as a vinyl greenhouse, they could cause more errors in detection. For those reasons, a rain sensor and a condensation sensor are considered inadequate in a case in which a relatively short-time detection is required and they are assumed to be used in a high temperature and humidity environment as in the present embodiment.

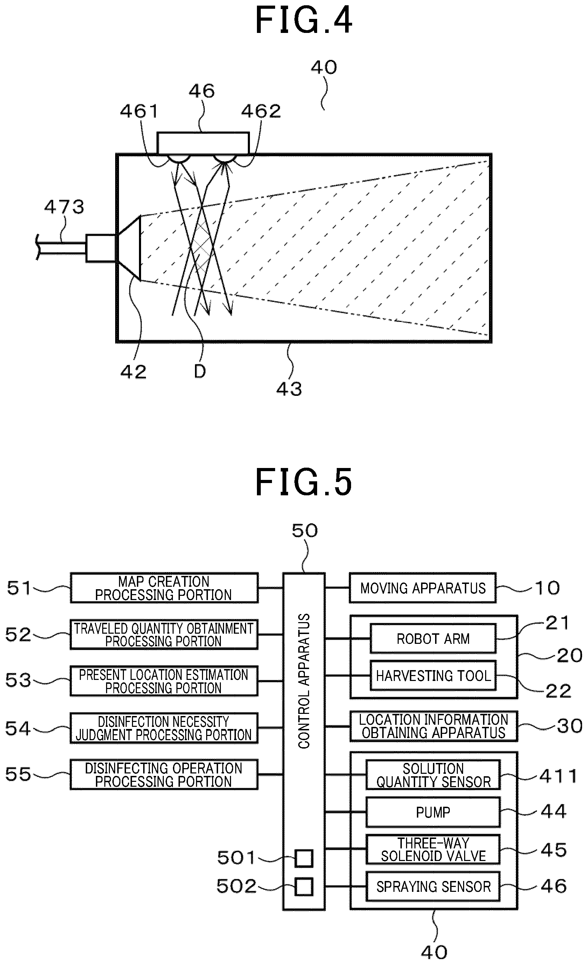

As a solution, in this embodiment, an optical sensor, a so-called optoelectronic sensor is used as the spraying sensor 46. In this case, the spraying sensor 46 is a reflection-type optoelectronic sensor and has a light-emitting portion 461 and a light-receptive portion 462, as illustrated in Fig. 4. The spraying sensor 46 emits light such as visible light or infrared light from the light-emitting portion 461 toward a spraying area of the spraying portion 42 and receives reflected light by the light-receptive portion 462. The spraying sensor 46 then detects the presence or absence of the disinfectant solution with reference to the difference in quantity between the emitted light and the received light. In this case, a mesh-patterned part in Fig. 4 indicates a detection area D of the spraying sensor 46. The spraying sensor 46 is adjusted such that the disinfectant solution sprayed from the spraying portion 42 enters the detection area D of the spraying sensor 46.

Compared to pressure sensors, flow sensors, rain sensors, or condensation sensors, optical sensors respond very quickly because: light is fast; and a sensor circuit is fully composed of electronic parts and does not need time for mechanical operations. Furthermore, optical sensors can detect the disinfectant solution in a contactless manner. Consequently, they are hardly affected by ambient humidity, condensation, or the like and do not need to wait until they are ready to be used again as rain sensors or condensation sensors do.

In the present embodiment, by an optical sensor adopted as the spraying sensor 46, the state of spraying of the disinfectant solution will be able to be detected accurately, quickly, and almost in real time. Since the state of spraying of the disinfectant solution is detected accurately, quickly, and almost in real time, the crop harvesting system 1 achieves a precise control of the spraying of the disinfectant solution, resulting in a minimization of the quantity of the consumed disinfectant solution.

The disinfecting apparatus 40 should not be limited to an apparatus that performs disinfection by spraying a disinfectant solution. For example, the disinfecting apparatus 40 may be configured to perform disinfection by soaking the blade part 221 of the harvesting tool 22, which is a disinfection target part, in a container filled with the disinfectant solution. Alternatively, the disinfecting apparatus 40 can adopt a method of: bringing the blade part 221 of the harvesting tool 22 into contact with a fabric-like object impregnated with the disinfectant solution; applying disinfectant hot blast or water of 60 degrees or higher, for example, to the blade part 221 of the harvesting tool 22; bringing the blade part 221 of the harvesting tool 22 near to a heating element; or emitting sterilizing light such as ultraviolet light to the blade part 221 of the harvesting tool 22.

For example, the control apparatus 50 is mainly composed of a microcomputer having a CPU 501, a ROM, a RAM, and a rewritable storage area 502 such as a flash memory, as illustrated in Fig. 5. The control apparatus 50 controls driving of the moving apparatus 10, the harvesting apparatus 20, the location information obtaining apparatus 30, and the disinfecting apparatus 40. For example, the control apparatus 50 may be connected with the higher-level apparatus 60 via a telecommunication line 70 such as a LAN, a WAN, the internet, or a cellular network so that it can communicate with the higher-level apparatus 60, as illustrated in Fig. 1. In this case, for example, the higher-level apparatus 60 may be installed within or outside the farm 90. Alternatively, the higher-level apparatus 60 may be connected directly with the control apparatus 50 by a cable or the like.

For example, the higher-level apparatus 60 is a server, a personal computer, or the like and has an input portion 61 and an output portion 62. For example, the input portion 61 is a user interface such as a keyboard, a mouse, a touch-screen panel, or the like. For example, the output portion 62 is a display, a speaker, or the like. Using the input portion 61 and the output portion 62, a user can remotely manipulate the crop harvesting system 1 via the telecommunication line 70.

The storage area 502 illustrated in Fig. 5 records programs for the crop harvesting system. The control apparatus 50 executes the programs for the crop harvesting system by the CPU 501. Thereby, a map creation processing portion 51, a traveled quantity obtainment processing portion 52, a present location estimation processing portion 53, a disinfection necessity judgment processing portion 54, a disinfecting operation processing portion 55, and the like are virtually realized by software. Alternatively, the control apparatus 50, the map creation processing portion 51, the traveled quantity obtainment processing portion 52, the present location estimation processing portion 53, the disinfection necessity judgment processing portion 54, and the disinfecting operation processing portion 55 may be unified into an integrated circuit, for example, and realized in terms of hardware.

The map creation processing portion 51 may perform a map creating process. The map creating process includes a process of creating, in data, a map of the farm 90 illustrated in Fig. 6, including the harvesting operation area 901 and the connecting area 902, in accordance with input by a worker. In this case, the worker can create a map using the input portion 61 of the higher-level apparatus 60. The map creation processing portion 51 is further allowed to download to the control apparatus 50 the data of an externally created map of the farm 90 via the telecommunication line 70 or an electric wiring.

In a case in which the location information obtaining apparatus 30 is a visual apparatus that is can measure a three-dimensional space or object, such as a stereo camera, a ToF camera, a structured-light scanner, or the like, the map creation processing portion 51 will be able to create a map of the farm 90 in accordance with visual information obtained from the location information obtaining apparatus 30 which is a visual apparatus. ToF is short for Time of Flight. The map creation processing portion 51 can create a map of the farm 90 in accordance with visual nearby information obtained from the location information obtaining apparatus 30 while the moving apparatus 10 is moving in the farm 90. In this case, the moving apparatus 10 may move in the farm 90 autonomously in accordance with instructions from the control apparatus 50 or the higher-level apparatus 60, or may move in the farm 90, for example, by being manipulated by a user from the input portion 61 of the higher-level apparatus 60.

The traveled quantity obtainment processing portion 52 may perform a traveled quantity obtaining process. The traveled quantity obtaining process includes a process of obtaining the quantity traveled by the moving apparatus 10. In this case, the traveled quantity obtainment processing portion 52 can be configured to receive measured results from an encoder that measures the number of rotations of a driving motor of the moving apparatus 10, measure the number of rotations of the wheel 11, and measure the quantity i.e., distance traveled by the moving apparatus 10 with reference to the number of rotations of the wheel 11. The traveled quantity obtainment processing portion 52 may obtain the quantity traveled by the moving apparatus 10 anytime while the moving apparatus 10 is moving. The quantity traveled by the moving apparatus 10, which is obtained by the traveled quantity obtaining process, is recorded, for example, in the storage area 502 or the like.

The present location estimation processing portion 53 may perform a present location estimating process. The present location estimating process includes a process of estimating the present location of the moving apparatus 10 with reference to the location information obtained by the location information obtaining apparatus 30. In the case of the present embodiment, the present location estimation processing portion 53 includes a process of estimating the present location of the moving apparatus 10 with reference to the map of the farm 90 including the harvesting operation area 901 and the connecting area 902, which is created by the map creation processing portion 51. In other words, the present location estimation processing portion 53 may estimate where on the map created by the map creation processing portion 51, the moving apparatus 10 is presently located, with reference to the present location information of the moving apparatus 10, which is obtained by the location information obtaining apparatus 30.

In the case of the present embodiment, the present location estimation processing portion 53 further includes a process of estimating the present location of the moving apparatus 10 with reference to the quantity traveled by the moving apparatus 10 from a reference position, which is obtained by the traveled quantity obtainment processing portion 52. The reference position can be set at any desired point, for example, on the route of the moving apparatus 10; it may be a fixed point or a point at which the location information is obtained by the location information obtaining apparatus 30. For example, the present location estimation processing portion 53 sets in advance the route for the moving apparatus 10 to move along, on the map created by the map creation processing portion 51. The present location estimation processing portion 53 can thus estimate the present location of the moving apparatus 10 as a point on the route set on the map, which is away from the reference position by the traveled quantity obtained by the traveled quantity obtainment processing portion 52.

The present location estimation processing portion 53 may use both of: a method of estimating the present location of the moving apparatus 10 with reference to the location information obtained by the location information obtaining apparatus 30; and a method of estimating the present location of the moving apparatus 10 with reference to the quantity traveled by the moving apparatus 10, which is obtained by the traveled quantity obtainment processing portion 52. This means, in a case in which it is configured such that the signs 94 and 95 are installed on the actual route, the location information obtaining apparatus 30 would not be able to obtain the present location of the moving apparatus 10 from a section in which the signs 94 and 95 are not installed. In another case in which the location information obtaining apparatus 30 is configured to obtain the location information using a GPS, the location information obtaining apparatus 30 would not be able to obtain the present location of the moving apparatus 10 at any location at which GPS radio waves cannot be received because of structures in the farm 90. In yet another case in which the present location estimation processing portion 53 is configured to estimate the present location of the moving apparatus 10 with reference to the quantity traveled by the moving apparatus 10, the present location estimation processing portion 53 would be able to successively estimate the present location of the moving apparatus 10, possibly causing more errors in the estimation.

As a solution, for example, when the location information obtaining apparatus 30 is at a point at which the signs 94 and 95 are installed or at a point at which GPS radio waves can be received, the present location estimation processing portion 53 estimates the present location of the moving apparatus 10 using the signs 94 and 95 read by the location information obtaining apparatus 30 or using GPS radio waves received by the location information obtaining apparatus 30. When the location information obtaining apparatus 30 is in a section in which the signs 94 and 95 are not installed or in a section in which GPS radio waves cannot be received, the present location estimation processing portion 53 estimates the present location of the moving apparatus 10 with reference to the quantity traveled by the moving apparatus 10, which is obtained by the traveled quantity obtainment processing portion 52. This means, these methods serve as a complement to each other for providing complete information. The present location estimation processing portion 53 can thus estimate the present location of the moving apparatus 10 accurately.

The disinfection necessity judgment processing portion 54 may perform a disinfection necessity judgment process. The disinfection necessity judgment process is a process of judging whether disinfection to a disinfection target part such as the blade part 221 of the harvesting tool 22 is necessary. The disinfection necessity judgment process includes a process of judging that disinfection to a disinfection target part such as the blade part 221 of the harvesting tool 22 is necessary in a case in which the present location estimation processing portion 53 judges that the moving apparatus 10 is adjacent to the harvesting operation area 901. In the case of the present embodiment, the disinfection necessity judgment processing portion 54 judges that disinfection to the blade part 221 or the like of the harvesting tool 22, which is a disinfection target part, is necessary in a case in which the moving apparatus 10 is about to enter the harvesting operation area 901 from an area outside the harvesting operation area 901, i.e., in a case in which the moving apparatus 10 is moving toward the harvesting operation area 901. Specifically, for example, the disinfection necessity judgment processing portion 54 may judge that disinfection is necessary in a case in which the moving apparatus 10 moves toward the harvesting operation area 901 from the trace line 93. More specifically, for example, the disinfection necessity judgment processing portion 54 may judge that disinfection is necessary in a case in which the moving apparatus 10 detects the sign 95 which is installed at a branch point of the trace line 93, then approaches the harvesting operation area 901 along a branch line, further moves toward the harvesting operation area 901, then detects the sign 94 which is installed adjacent to the harvesting operation area 901.

The disinfecting operation processing portion 55 performs a disinfecting operation when it is judged in the disinfection necessity judgment process that disinfection is necessary. In the case of the present embodiment, the disinfecting operation processing portion 55 may perform a disinfecting operation promptly when it is judged in the disinfection necessity judgment process that disinfection is necessary. The disinfecting operation is an operation of disinfecting the blade part 221 of the harvesting tool 22, which is a disinfection target part, by driving at least one of the harvesting apparatus 20 and the disinfecting apparatus 40. For example, in a case in which the disinfecting apparatus 40 is configured to perform disinfection by soaking the blade part 221 in a disinfectant tub filled with the disinfectant solution or by bringing the blade part 221 into contact with fabric or the like impregnated with the disinfectant solution, the disinfecting operation processing portion 55 will disinfect the blade part 221 by driving the robot arm 21 of the harvesting apparatus 20.

In another case in which the disinfecting apparatus 40 is configured to spray the disinfectant solution to the blade part 221 by driving the pump 44 or the like as in the present embodiment, the disinfecting operation processing portion 55 will disinfect the blade part 221 by driving the robot arm 21 of the harvesting apparatus 20 and the disinfecting apparatus 40.

For example, in yet another case in which the initial position of the robot arm 21 is set in advance such that the blade part 221 is retracted in the covering member 43, and the harvesting apparatus 20 is not in operation for harvesting, the blade part 221 may be retracted in the covering member 43. In this case, the disinfecting operation processing portion 55 will not need bringing the blade part 221 of the harvesting tool 22 into the covering member 43 by driving the robot arm 21, to perform a disinfecting operation. In other words, in this case, the disinfecting operation processing portion 55 will be able to disinfect the blade part 221 by driving the disinfecting apparatus 40 without driving the harvesting apparatus 20.

Alternatively, the disinfecting operation processing portion 55 may perform a disinfecting operation while the moving apparatus 10 remains in the connecting area 902. In other words, the disinfecting operation processing portion 55 can be configured not to perform a disinfecting operation while the moving apparatus 10 remains in the harvesting operation area 901, i.e., while a harvesting operation is taking place.

The harvesting apparatus 20 further makes the disinfection target part operate as in harvesting while the disinfecting apparatus 40 is in operation for disinfection. In the case of the present embodiment, making the blade part 221 of the harvesting tool 22, which is a disinfection target part, operate as in harvesting is an operation of opening and closing the scissors-shaped blade part 221. The harvesting apparatus 20 thus opens and closes the blade part 221 while the disinfecting apparatus 40 is in operation for disinfection, i.e., while the disinfectant solution is being sprayed from the spraying portion 42. This facilitates distribution of the disinfectant solution over the entire blade part 221 even to an in-between space and disinfection of the entire blade part 221.

The harvesting apparatus 20 further performs an operation of changing the posture of the harvesting tool 22 by driving the robot arm 21 while the disinfecting apparatus 40 is in operation for disinfection. This further facilitates distribution of the disinfectant solution over the entire blade part 221 and disinfection of the entire blade part 221 which is a disinfection target part.

### [Control Flow]

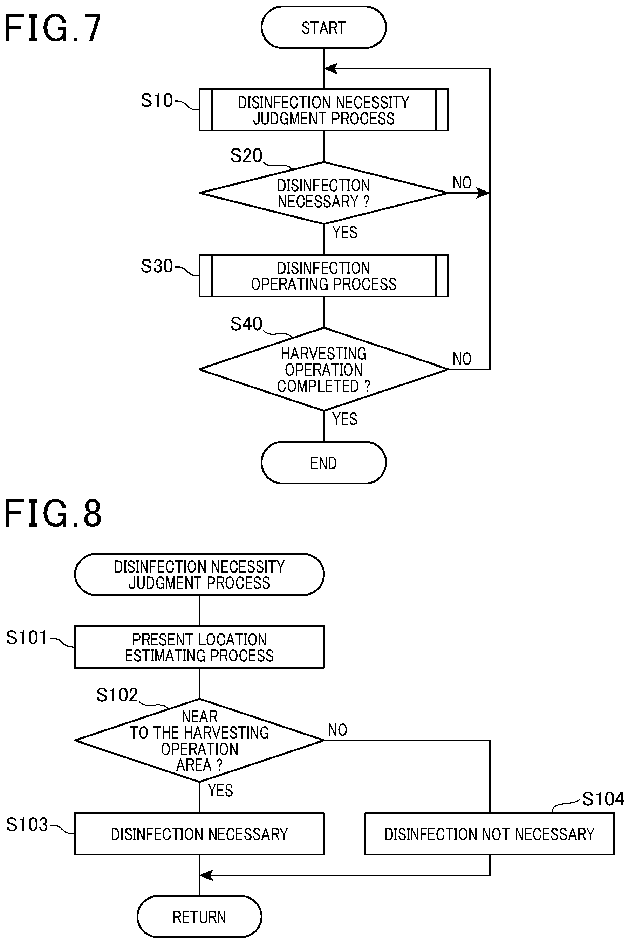

Hereinafter, an example of a series of control flows to be invoked by the crop harvesting system 1, which is related to a disinfecting operation, will be described with reference to Figs. 7 to 9 as well. In the following description, the agent of a process to be perform in each step will be the control apparatus 50. In the example of Fig. 6, the moving apparatus 10 moves along the rail 92 in the harvesting operation area 901 and moves along the trace line 93 in the connecting area 902. When the moving apparatus 10 enters the harvesting operation area 901, the harvesting apparatus 20 mounted on the moving apparatus 10 sequentially or selectively harvests the harvesting objects T cultivated in the cultivating device 911. Upon completion of a harvesting operation in one the harvesting operation area 901 in which the moving apparatus 10 is currently present, the moving apparatus 10 leaves this harvesting operation area 901 and moves to another harvesting operation area 901 by way of the connecting area 902.

In this configuration, the disinfecting apparatus 40 disinfects the blade part 221 of the harvesting tool 22, which is a disinfection target part, while the moving apparatus 10 is located adjacent to the harvesting operation area 901 and remains in the connecting area 902. In this case, the control apparatus 50 invokes a series of the control flows related to a disinfecting operation (Start of Fig. 7), then performs a disinfection necessity judgment process in Step S10. Upon performing the disinfection necessity judgment process, the control apparatus 50 performs a location estimating process in Step S101 of Fig. 8 to estimate the present location of the moving apparatus 10.

Subsequently, in Step S102, the control apparatus 50 judges whether the present location of the moving apparatus 10, which is estimated in Step S101, is adjacent to the harvesting operation area 901. When the present location of the moving apparatus 10 is adjacent to the harvesting operation area 901 (YES in Step S102), the control apparatus 50 moves the procedure to Step S103 to judge that disinfection is necessary, then moves the procedure to Step S20 of Fig. 7 (Return of Fig. 8). When the present location of the moving apparatus 10 is not adjacent to the harvesting operation area 901 (NO in Step S102), i.e., when the present location of the moving apparatus 10 is in the harvesting operation area 901, the control apparatus 50 moves the procedure to Step S104 to judge that disinfection is not necessary, then returns the procedure to the flow of Fig. 7 (Return of Fig. 8).

Subsequently, in Step S20 of Fig. 7, the control apparatus 50 judges the results from the disinfection necessity judgment process of Step S10. When it is judged in the disinfection necessity judgment process of Step S10 that disinfection is not necessary (NO in Step S20), the control apparatus 50 returns the procedure to Step S10 to repeat the procedure from Step S10. When it is judged in the disinfection necessity judgment process of Step S10 that disinfection is necessary (YES in Step S20), the control apparatus 50 performs a disinfection operating process in Step S30.

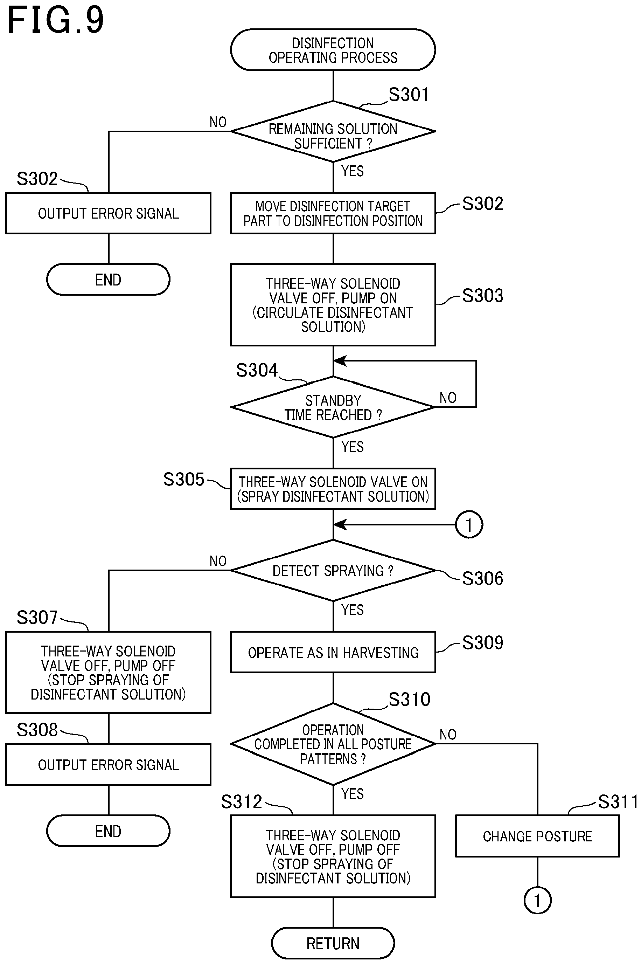

As shown in Fig. 9, upon performing the disinfection operating process in Step S30, the control apparatus 50 judges in Step S301 whether the quantity of the remaining solution in the disinfectant solution tank 41 is sufficient, with reference to detection results from the solution quantity sensor 411. When the quantity of the remaining disinfectant solution stored in the disinfectant solution tank 41 performs short (NO in Step S301), the control apparatus 50 moves the procedure to Step S302. Subsequently, in Step S302, the control apparatus 50 outputs to the higher-level apparatus 60, for example, an error signal that notifies that the quantity of the remaining disinfectant solution stored in the disinfectant solution tank 41 is insufficient. In this case, for example, the disinfecting apparatus 40 encourages nearby workers to add the disinfectant solution, by emitting an error sound from a speaker or the like of the disinfecting apparatus 40. Receiving the error signal, the higher-level apparatus 60 encourages a supervisor or the like to add the disinfectant solution, by emitting an error sound from the output portion 62 or displaying an error alert or the like on the output portion 62. The control apparatus 50 then terminates the procedure for now (End).

When the quantity of the remaining disinfectant solution stored in the disinfectant solution tank 41 is sufficient (YES in Step S301), the control apparatus 50 continues the procedure from Step S302. In this case, in Step S302, the control apparatus 50 moves the disinfection target part to a disinfection position by driving the harvesting apparatus 20. In the case of the present embodiment, the disinfection target part is the blade part 221 of the harvesting tool 22, and the disinfection position is inside the covering member 43. So, as referred to Fig. 3, the control apparatus 50 inserts at least the blade part 221 of the harvesting tool 22 into the covering member 43 through the opening portion 431 of the covering member 43, by driving the robot arm 21 of the harvesting apparatus 20.

Subsequently, in Step S303, the control apparatus 50 switches the pump 44 to the ON state while keeping the three-way solenoid valve 45 in the OFF state. The disinfectant solution in the disinfectant solution tank 41 is sucked by the pump 44, conveyed by way of the three-way solenoid valve 45, and returned to the disinfectant solution tank 41. The disinfectant solution is thus circulated by way of the disinfectant solution tank 41, the pump 44, and the three-way solenoid valve 45.

Subsequently, in Step S303, the control apparatus 50 judges whether a predetermined standby time has elapsed since the pump 44 is switched to the ON state. The predetermined standby time is a period that is determined to be sufficient to, by driving the pump 44, expel any trapped air from the piping 471, which connects the disinfectant solution tank 41 and the pump 44, and the piping 472, which connects the pump 44 and the three-way solenoid valve 45, and fill them with the disinfectant solution. The standby time is determined depending on the capacity of the pump 44, the volumetric capacity of the pipings 471 and 472, and the like. In the case of the present embodiment, it is determined to be, for example, roughly 10 seconds to 1 minute.

When the predetermined standby time has elapsed since Step S303 in which the pump 44 is switched to the ON state, the pipings 471 and 472 of the spraying route J composed of the pipings 471, 472, and 473, which connect, respectively, the disinfectant solution tank 41 to the pump 44 and the pump 44 to the three-way solenoid valve 45, become filled with the disinfectant solution. This causes an increase in the pressure in the piping 472.

When the standby time has not elapsed yet (NO in Step S304), the control apparatus 50 waits until the standby time has elapsed, by repeating the Step S304. When the standby time has elapsed (YES in Step S304), the control apparatus 50 moves the procedure to Step S305 to switch the three-way solenoid valve 45 to the ON state while keeping the pump 44 in the ON state. The flow of the disinfectant solution is thus switched from the return route R to the spraying route J, and the disinfectant solution is sprayed from the spraying portion 42. The process of Step S303 and the processes of Step S304 and S305 may be perform at the same time, or the order may be changed.

Subsequently, in Step S306, the control apparatus 50 judges whether the disinfectant solution is being sprayed from the spraying portion 42, with reference to detection results by the spraying sensor 46. When the spraying sensor 46 does not detect spraying of the disinfectant solution from the spraying portion 42 (NO in Step S306), the control apparatus 50 moves the procedure to Step S307. The control apparatus 50 further stops spraying of the disinfectant solution by switching both the three-way solenoid valve 45 and the pump 44 to the OFF state.

After that, in Step S308, the control apparatus 50 outputs to the higher-level apparatus 60, for example, an error signal that notifies of an abnormality in spraying of the disinfectant solution from the spraying portion 42. In this case, for example, the disinfecting apparatus 40 encourages nearby workers to conduct maintenance of the disinfecting apparatus 40, by emitting an error sound from a speaker or the like of the disinfecting apparatus 40. Receiving the error signal, the higher-level apparatus 60 encourages a supervisor or the like to conduct maintenance of the disinfecting apparatus 40, by emitting an error sound from the output portion 62 or displaying an error alert or the like on the output portion 62. The control apparatus 50 then terminates the procedure for now (End).

When the spraying sensor 46 detects spraying of the disinfectant solution from the spraying portion 42 (YES in Step S306), the control apparatus 50 moves the procedure to Step S309. In Step S309, while the disinfectant solution is being sprayed from the spraying portion 42, the control apparatus 50 makes the harvesting tool 22 perform the same operation as in harvesting, by driving the harvesting tool 22. In the case of the present embodiment, in Step S309, the control apparatus 50 makes the harvesting tool 22 open and close the blade part 221. This facilitates distribution of the disinfectant solution over the entire blade part 221 even to an in-between space and disinfection of the entire blade part 221 which is a disinfection target part.

After that, the control apparatus 50 judges whether disinfection is complete for all preset posture patterns. In this case, a posture pattern is the posture of the harvesting tool 22 being turned in a pitching direction, a rolling direction, or a yawing direction. In the case of the present embodiment, the control apparatus 50 performs a disinfecting operation for two or more different posture patterns. When the disinfecting operation is complete in all the posture patterns (NO in Step S310), the control apparatus 50 moves the procedure to Step S311. The control apparatus 50 changes the posture of the harvesting tool 22 by controlling the robot arm 21, then returns the procedure to Step S306. Thus, the control apparatus 50 performs a disinfecting operation again to the harvesting tool 22 which is now in a changed posture.

When the disinfecting operation is complete in all the preset posture patterns (YES in Step S310), the control apparatus 50 moves the procedure to Step S312. In Step S312, the control apparatus 50 stops spraying of the disinfectant solution by switching the three-way solenoid valve 45 to the OFF state and the pump 44 to the OFF state. The control apparatus 50 then moves the procedure to Step S40 of Fig. 7 (Return of Fig. 9).

In Step S40, the control apparatus 50 judges whether the harvesting operation is complete by the crop harvesting system 1. When the harvesting operation is not complete (NO in Step S40), the control apparatus 50 returns the procedure to Step S10 to repeat the procedure from Step S10. When the harvesting operation is complete (YES in Step S40), the control apparatus 50 terminates the series of the control flows related to a disinfecting operation (End).

According to the above-described embodiment, the crop harvesting system 1 is provided with the moving apparatus 10, the harvesting apparatus 20, and the disinfecting apparatus 40. The moving apparatus 10 is configured to be movable in the harvesting operation area 901 which is an area in which the harvesting operation takes place. The harvesting apparatus 20 is mounted on the moving apparatus 10 and has a function of harvesting the crops T, which are harvesting objects, in contact with the harvesting objects T. The disinfecting apparatus 40 has a function of disinfecting at least the disinfection target part 221 of the harvesting apparatus 20, which is to be brought into contact with the harvesting objects T. In the case of the present embodiment, the disinfecting apparatus 40 has a function of disinfecting at least the blade part 221 of the harvesting tool 22.

The crop harvesting system 1 is provided with the disinfection necessity judgment processing portion 54 and the disinfecting operation processing portion 55. The disinfection necessity judgment processing portion 54 may perform a disinfection necessity judgment process of judging whether disinfection of the disinfection target part 221, which is the blade part 221 of the harvesting tool 22 in this case, is necessary. When it is judged in the disinfection necessity judgment process that disinfection is necessary, the disinfecting operation processing portion 55 can perform a disinfecting operation of disinfecting the disinfection target part 221, which is the blade part 221 of the harvesting tool 22 in this case, by driving at least one of the harvesting apparatus 20 and the disinfecting apparatus 40.

According to this crop harvesting system 1, at least the blade part 221 or the like of the harvesting tool 22, for example, which is to be brought into direct contact with the harvesting objects T, will be able to be automatically disinfected. Since workers do not need to manually disinfect the harvesting apparatus 20, disinfecting operations will require less effort than in a case in which they manually disinfect the harvesting apparatus 20, which results in an increase in the operating efficiency in harvesting operations. Furthermore, workers will not accidentally forget about disinfecting it as in the case in which they manually disinfect the harvesting apparatus. Consequently, even in a case in which one individual object in the farm 90 is affected with a disease, the spread of the disease all over the farm 90 by the medium of the harvesting tool 22 will be able to be reduced to a minimum.

The crop harvesting system 1 is further provided with the location information obtaining apparatus 30 and the present location estimation processing portion 53. The location information obtaining apparatus 30 can obtain present location information of the moving apparatus 10. The present location estimation processing portion 53 may perform a present location estimating process. The present location estimating process includes a process of estimating the present location of the moving apparatus 10 with reference to the location information obtained by the location information obtaining apparatus 30. The disinfection necessity judgment process includes a process of judging that a disinfecting operation by the disinfecting apparatus 40 is necessary in a case in which it is judged that the harvesting apparatus 20 is going to start a harvesting operation, with reference to the present location of the moving apparatus 10, which is estimated in the present location estimating process. The disinfecting operation processing portion 55 performs a disinfecting operation before the harvesting apparatus 20 performs a harvesting operation.

Accordingly, the disinfecting operation processing portion 55 performs a disinfecting operation to the disinfection target part 221, which is the blade part 221 of the harvesting tool 22 in this case, before the harvesting apparatus 20 performs a harvesting operation. So, the spread of the disease all over the farm 90 by the medium of the harvesting tool 22 will be able to be more effectively reduced.

In this case, the disinfection necessity judgment process includes a process of judging that a disinfecting operation by the disinfecting apparatus 40 is necessary in a case in which the moving apparatus 10 is about to enter the harvesting operation area 901, i.e., in a case in which the moving apparatus 10 is moving toward the harvesting operation area 901. Thus, the disinfection target part, which is the blade part 221 of the harvesting tool 22 in this case, will be able to be regularly disinfected in a case in which the moving apparatus 10 moves to another harvesting operation area 901 to perform a harvesting operation. Consequently, the spread of the disease throughout the farm 90 by the medium of the harvesting tool 22 will be able to be even more effectively reduced.

The disinfecting operation processing portion 55 makes the disinfecting apparatus 40 perform a disinfecting operation while the present location of the moving apparatus 10, which is estimated in the present location estimating process, is outside the harvesting operation area 901. This means, the disinfecting apparatus 40 performs a disinfecting operation to the blade part 221 of the harvesting tool 22, which is a disinfection target part, while the moving apparatus 10 remains in an area outside the harvesting operation area 901. In other words, the disinfecting apparatus 40 does not perform a disinfecting operation to the blade part 221 of the harvesting tool 22, which is a disinfection target part, while the moving apparatus 10 remains in the harvesting operation area 901, i.e., while a harvesting operation is taking place. In short, harvesting operations by the harvesting apparatus 20 will not be interfered with by disinfecting operations by the disinfecting apparatus 40. Consequently, a reduction in the operating efficiency in harvesting operations, which is due to disinfecting operations, will be able to be prevented.

The location information obtaining apparatus 30 obtains the present location of the moving apparatus 10 by a method including at least one of an RFID, a two-dimensional code, a magnetic marker, a visual apparatus, and a GPS. Thus, the location information obtaining apparatus 30 can obtain present location information of the moving apparatus 10 more accurately.

The crop harvesting system 1 is further provided with the map creation processing portion 51. The map creation processing portion 51 may perform a map creating process. The map creating process includes a process of creating a map of the harvesting operation area 901 in accordance with input by a worker or visual information obtained from a visual apparatus mounted on the moving apparatus 10, such as a camera or the like. The present location estimating process includes a process of estimating the present location of the moving apparatus 10 with reference to the map of the harvesting operation area 901 created in the map creating process. Thus, the present location estimation processing portion 53 can thus estimate the present location of the moving apparatus 10 more accurately. This allows the configuration of controlling a disinfecting operation with reference to the location of the moving apparatus 10 to achieve precise control of the timing for the disinfecting operation.

The crop harvesting system 1 is further provided with the traveled quantity obtainment processing portion 52. The traveled quantity obtainment processing portion 52 may perform a traveled quantity obtaining process of obtaining the quantity traveled by the moving apparatus 10. The present location estimating process further includes a process of estimating the present location of the moving apparatus 10 with reference to the quantity traveled by the moving apparatus 10 from a reference position. In other words, the present location estimation processing portion 53 estimates the present location of the moving apparatus 10 with reference to the location information obtained from the location information obtaining apparatus 30 and estimate the present location of the moving apparatus 10 with reference to the quantity traveled by the moving apparatus 10 as well.

Accordingly, even in a case which the moving apparatus 10 is in a section in which the location information obtaining apparatus 30 cannot obtain location information, the present location estimation processing portion 53 will be able to estimate the present location of the moving apparatus 10 with reference to the quantity traveled by the moving apparatus 10, which is obtained by the traveled quantity obtainment processing portion 52. So, the present location of the moving apparatus 10 will be estimated more accurately. This allows the configuration of controlling a disinfecting operation with reference to the location of the moving apparatus 10 to achieve a more precise control of the timing for the disinfecting operation.

The disinfecting apparatus 40 has the spraying portion 42 that ejects the disinfectant solution. The disinfecting apparatus 40 performs disinfection by spraying the disinfectant solution to the blade part 221 of the harvesting tool 22, which is a disinfection target part, from the spraying portion 42. Since liquid disinfectant solution is thus sprayed to the blade part 221, good efficiency in disinfection of the entire blade part 221 will be achieved.

The disinfecting apparatus 40 further has the optical spraying sensor 46 which may detect spraying of the disinfectant solution. This is affected by an external environment such as humidity less than in a case in which a pressure sensor, a flow sensor, a rain sensor, or a condensation sensor is adopted to detect spraying of the disinfectant solution. So, the state of spraying of the disinfectant solution will be able to be detected accurately, quickly, and almost in real time. Since the state of spraying of the disinfectant solution is detected accurately, quickly, and almost in real time, the crop harvesting system 1 achieves precise control of the spraying of the disinfectant solution, resulting in a reduction in the quantity of the consumed disinfectant solution.

The disinfecting apparatus 40 further has the covering member 43. The covering member 43 has the opening portion 431 which allows the blade part 221 of the harvesting tool 22, which is a disinfection target part, to be inserted therethrough. The covering member 43 has at least one lateral face that faces the direction in which the disinfectant solution is sprayed from the spraying portion 42.

Accordingly, a major part of the disinfectant solution sprayed from the spraying portion 42 does not hit the harvesting tool 22, which is a disinfection target part, but hits the covering member 43 which is set in position such that it faces the spraying portion 42. So, the disinfectant solution, which is sprayed from the spraying portion 42, will be prevented from scattering in all directions. Furthermore, in the case of the present embodiment, since the covering member 43, apart from the opening portion 431, encloses the spraying portion 42, the covering member 43 can prevent the disinfectant solution, which is sprayed from the spraying portion 42, from being carried away by the wind. Consequently, the disinfectant solution, which is sprayed from the spraying portion 42, will be able to hit the blade part 221 of the harvesting tool 22, which is a disinfection target part.

The disinfecting apparatus 40 has the disinfectant solution tank 41, the pump 44, and the three-way solenoid valve 45. The disinfectant solution tank 41 serves to store disinfectant solution. The pump 44 has a function of sucking up and spraying the disinfectant solution stored in the disinfectant solution tank 41. The three-way solenoid valve 45 has a function of selectively switching between the spraying route J, which connects the pump 44 and the spraying portion 42 to feed to the spraying portion 42 the disinfectant solution sprayed from the pump 44, and the return route R, which connects the pump 44 and the disinfectant solution tank 41 to return to the disinfectant solution tank 41 the disinfectant solution sprayed from the pump 44.

To perform a disinfecting operation, the disinfecting apparatus 40 circulates the disinfectant solution for a predetermined period by way of the disinfectant solution tank 41 and the pump 44, by driving the pump 44 while the three-way solenoid valve 45 is on the return route R. After that, the disinfecting apparatus 40 ejects the disinfectant solution from the spraying portion 42 by switching the three-way solenoid valve 45 to the spraying route J.

Accordingly, until the three-way solenoid valve 45 is switched to the spraying route J, any trapped air will be expelled from at least the pipings 471 and 472 of the spraying route J composed of the pipings 471, 472, and 473 and leading from the disinfectant solution tank 41 to the three-way solenoid valve 45, and these pipings will be filled with the disinfectant solution. This causes an increase in the pressure in the piping 472. When the three-way solenoid valve 45 is switched to the ON state to open the spraying route J, the disinfectant solution will be immediately sprayed from the spraying portion 42. This enables a precise control of the spraying of the disinfectant solution, shortening of the duration of spraying, and minimization of the quantity of the consumed disinfectant solution.

The disinfecting apparatus 40 is mounted on the moving apparatus 10 and configured to be movable along with the moving apparatus 10. Thus, the moving apparatus 10 does not need to move to an installation location of the disinfecting apparatus 40 as in a case in which the disinfecting apparatus 40 is installed at a fixed location in the farm 90. The disinfecting apparatus 40 may perform a disinfecting operation while moving along with the moving apparatus 10. In other words, this configuration will eliminate the time for the moving apparatus 10 to move to the installation location of the disinfecting apparatus 40 for a disinfecting operation or the time for the moving apparatus 10 to stand by for a disinfecting operation. Consequently, disinfecting operations will be performed without reducing the operating ratio of the moving apparatus 10 including the harvesting apparatus 20 to the greatest extent possible.

While the disinfecting apparatus 40 is performing the disinfecting operation, the harvesting apparatus 20 makes the disinfection target part operate as in harvesting. In the case of the present embodiment, the harvesting apparatus 20 opens and closes the blade part 221 of the harvesting tool 22. This facilitates distribution of the disinfectant solution over the entire blade part 221 even to an in-between space and disinfection of the entire blade part 221 which is a disinfection target part.

The harvesting apparatus 20 is provided with: the articulated robot arm 21; and the harvesting tool 22 which is attached to an arm end part of the robot arm 21 and to harvest the harvesting objects T in contact with the harvesting objects T. The harvesting apparatus 20 performs an operation of changing the posture of the harvesting tool 22 by driving the robot arm 21 while the disinfecting apparatus 40 is performing a disinfecting operation.

This further facilitates distribution of the disinfectant solution over the entire blade part 221 and disinfection of the entire blade part 221 which is a disinfection target part.

### (Second Embodiment)

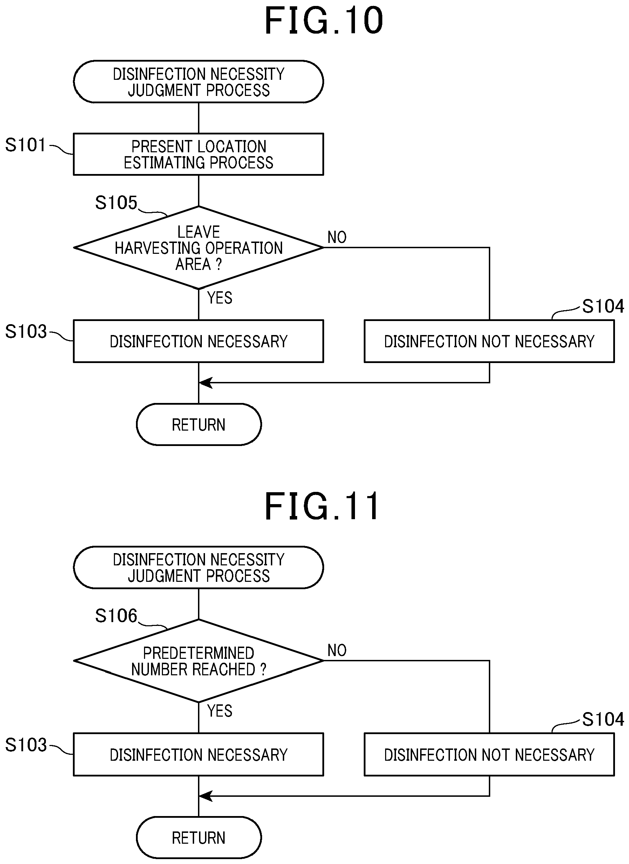

Hereinafter, a second embodiment will be described with reference to Fig. 10.

In the present embodiment, the specific mode of the disinfection necessity judgment process is different from in the first embodiment described above. That is, in the present embodiment, the disinfection necessity judgment process includes a process of judging that a disinfecting operation by the disinfecting apparatus 40 is necessary in a case in which the moving apparatus 10 leaves the harvesting operation area 901. In this case, the control apparatus 50 performs Step S105 of Fig. 10 instead of Step S102 of Fig. 8.

In Step S105, the control apparatus 50 judges whether the moving apparatus 10 leaves the harvesting operation area 901, with reference to the present location of the moving apparatus 10, which is estimated in Step S101. When the moving apparatus 10 leaves the harvesting operation area 901 (YES in Step S105), the control apparatus 50 moves the procedure to Step S103 to judge that a disinfecting operation is necessary. When the moving apparatus 10 does not leave the harvesting operation area 901 (NO in Step S105), the control apparatus 50 moves the procedure to Step S104 to judge that a disinfecting operation is not necessary.

This will produce the same effect as the first embodiment described above.

### (Third Embodiment)

Hereinafter, a third embodiment will be described with reference to Fig. 11.

Similarly, in the present embodiment, the specific mode of the disinfection necessity judgment process is different from in the above-described embodiments. That is, in the present embodiment, the disinfection necessity judgment process includes a process of judging whether a disinfecting operation by the disinfecting apparatus 40 is necessary, with reference to the number of the harvesting objects T having been harvested by the harvesting apparatus 20. In this case, the control apparatus 50 can exclude the present location of the moving apparatus 10 from the factors with reference to which the necessity of a disinfecting operation is judged. In this case, the control apparatus 50 does not perform Step S101 of Fig. 8 but performs Step S106 of Fig. 11 instead of Step S102 of Fig. 8.

In Step S106, the control apparatus 50 judges the number of the harvesting objects T having been harvested since the last disinfecting operation. When the number of the harvesting objects T having been harvested since the last disinfecting operation is a predetermined number or greater (YES in Step S106), the control apparatus 50 moves the procedure to Step S103 to judge that a disinfecting operation is necessary. When the number of the harvesting objects T having been harvested since the last disinfecting operation is less than the predetermined number (NO in Step S106), the control apparatus 50 moves the procedure to Step S104 to judge that a disinfecting operation is not necessary.

This further will produce the same effect as the above-described embodiments.

### (Fourth Embodiment)

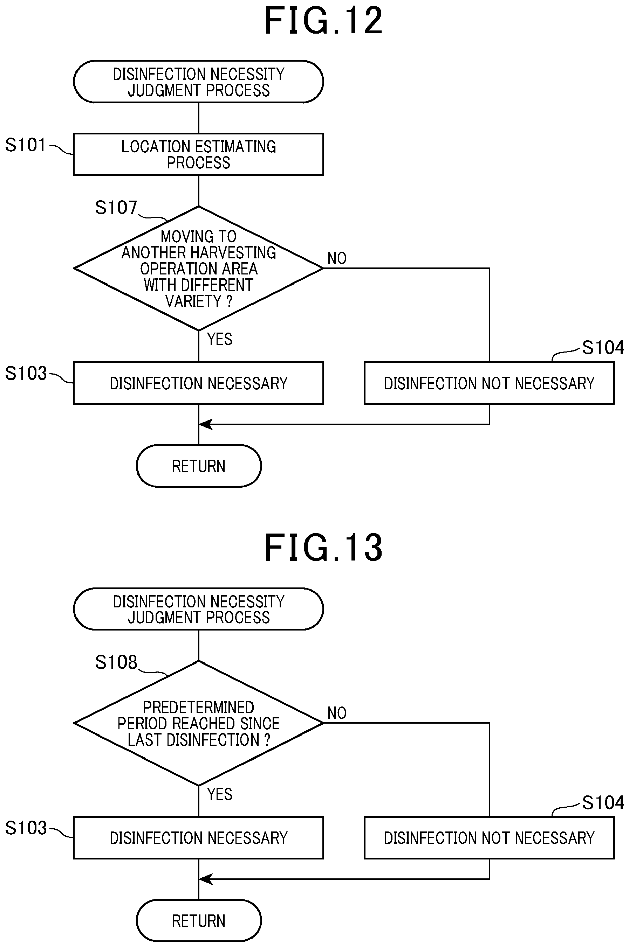

Hereinafter, a fourth embodiment will be described with reference to Fig. 12.

Similarly, in the present embodiment, the specific mode of the disinfection necessity judgment process is different from in the above-described embodiments. That is, in the present embodiment, the disinfection necessity judgment process includes a process of judging that a disinfecting operation by the disinfecting apparatus 40 is necessary in a case in which the moving apparatus 10 moves to another area in which the harvesting objects T of a different variety are cultivated. In this case, the control apparatus 50 performs Step S107 of Fig. 12 instead of Step S102 of Fig. 8.

In Step S107, the control apparatus 50 judges whether the moving apparatus 10 is moving to another harvesting operation area 901 in which the harvesting objects of a different variety are cultivated, with reference to the present location of the moving apparatus 10, which is estimated in Step S101. When the control apparatus 50 judges that the moving apparatus 10 is moving to another harvesting operation area 901 in which harvesting objects of a different variety are cultivated (YES in Step S107), the control apparatus 50 moves the procedure to Step S103 to judge that a disinfecting operation is necessary.

When the moving apparatus 10 is not moving to another harvesting operation area 901 in which the harvesting objects T of a different variety are cultivated (NO in Step S107), in other words, when the moving apparatus 10 is moving to another harvesting operation area 901 in which the harvesting objects of the same variety are cultivated, the control apparatus 50 moves the procedure to Step S104 to judge that a disinfecting operation is not necessary.

This further will produce the same effect as the above-described embodiments.

### (Fifth Embodiment)

Hereinafter, a fifth embodiment will be described with reference to Fig. 13.

Similarly, in the present embodiment, the specific mode of the disinfection necessity judgment process is different from in the above-described embodiments. That is, in the present embodiment, the disinfection necessity judgment process includes a process of judging whether a disinfecting operation by the disinfecting apparatus 40 is necessary, with reference to the time having lapsed since the last disinfecting operation performed by the disinfecting apparatus 40. In this case, the control apparatus 50 can exclude the present location of the moving apparatus 10 from the factors with reference to which the necessity of a disinfecting operation is judged. In this case, the control apparatus 50 does not perform Step S101 of Fig. 8, but performs Step S108 of Fig. 13 instead of Step S102 of Fig. 8.

In Step S108, the control apparatus 50 judges the time having elapsed since the last disinfecting operation. When a predetermined period has elapsed since the last disinfecting operation (YES in Step S108), the control apparatus 50 moves the procedure to Step S103 to judge that a disinfecting operation is necessary. When a predetermined period has not elapsed since the last disinfecting operation (NO in Step S108), the control apparatus 50 moves the procedure to Step S104 to judge that a disinfecting operation is no necessary.

This further will produce the same effect as the above-described embodiments.

### (Sixth Embodiment)

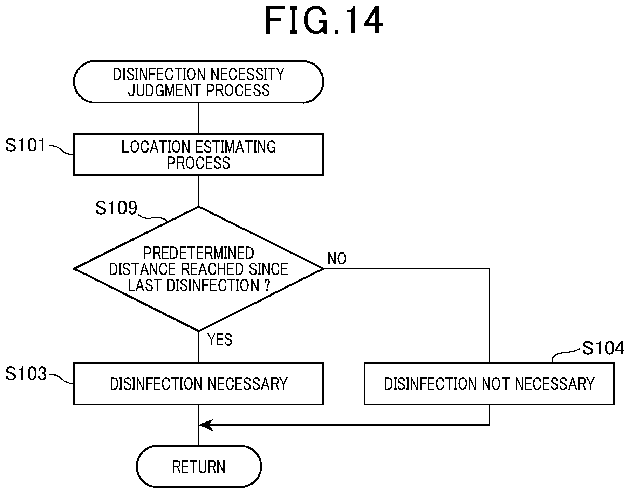

Hereinafter, a sixth embodiment will be described with reference to Fig. 14.

In the present embodiment, the specific mode of the disinfection necessity judgment process is different from in the above-described embodiments. That is, in the present embodiment, the disinfection necessity judgment process includes a process of judging whether a disinfecting operation by the disinfecting apparatus 40 is necessary, with reference to the quantity the moving apparatus 10 has moved since the last disinfecting operation is performed by the disinfecting apparatus 40. In this case, the control apparatus 50 performs Step S109 of Fig. 14 instead of Step S102 of Fig. 8.

In Step S109, the control apparatus 50 judges whether the quantity the moving apparatus 10 has moved from a point at which the last disinfecting operation took place reaches a predetermined distance, with reference to the present location of the moving apparatus 10, which is estimated in Step S101. When the quantity the moving apparatus 10 has moved from a point at which the last disinfecting operation took place reaches a predetermined distance (YES in Step S109), the control apparatus 50 moves the procedure to Step S103 to judge that a disinfecting operation is necessary. When the quantity the moving apparatus 10 has moved from a point at which the last disinfecting operation took place does not reach a predetermined distance (NO in Step S109), the control apparatus 50 moves the procedure to Step S104 to judge that a disinfecting operation is not necessary.

This further will produce the same effect as the first embodiment described above.

### (Seventh Embodiment)

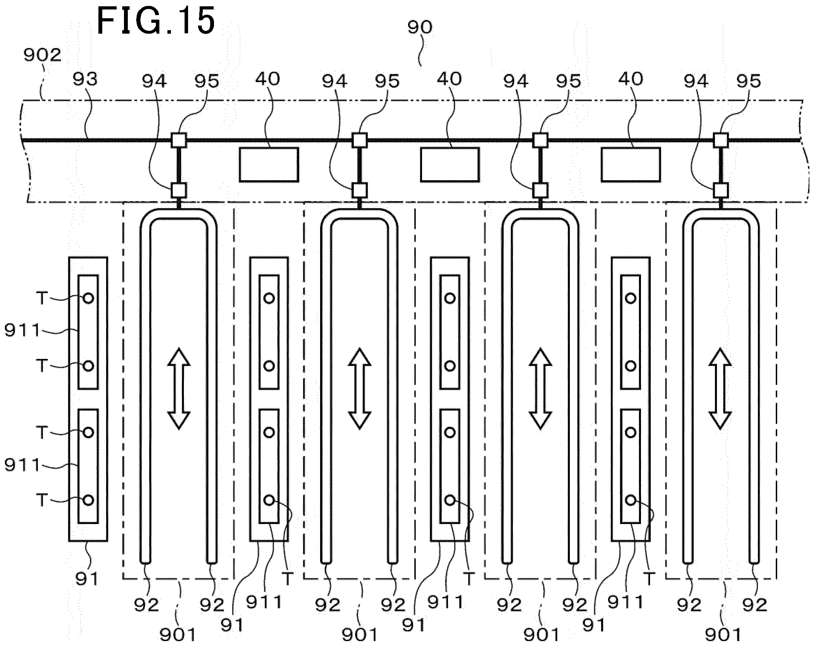

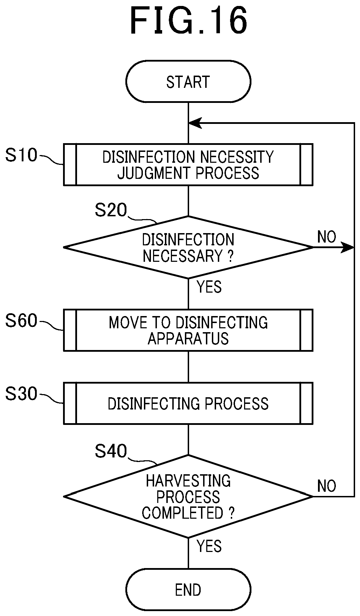

Hereinafter, a seventh embodiment will be described with reference to Figs. 15 and 16.

The present embodiment can be combined with any of the above-described embodiments. That is, in the present embodiment, the disinfecting apparatuses 40 are installed at specific locations in the farm 90, as illustrated in Fig. 15. The plurality of the disinfecting apparatuses 40 are installed in the connecting area 902, for example, on the route along which the moving apparatus 10 moves.

As shown in Fig. 16, when it is judged in Step S20 that a disinfecting operation is necessary, the control apparatus 50 performs the process of Step S60 to move the moving apparatus 10 to the disinfecting apparatus 40 installed in the farm 90. Subsequently, the control apparatus 50 performs Steps S30 and S40 as in the above-described embodiments,

This further will produce the same effect as the first embodiment described above.

Furthermore, this allows a plurality of the harvesting apparatuses 20 to use the disinfecting apparatus 40 on a shared basis. For example, in a case in which a lot of the moving apparatuses 10 are used together in the farm 90, each of the moving apparatuses 10 does not need to be equipped with the disinfecting apparatus 40. This will be able to minimize the number of the disinfecting apparatuses 40.

### (Other Embodiments)

The above-described embodiments show a configuration in which a disinfecting operation takes place before or after the harvesting apparatus 20 performs a harvesting operation. Alternatively, a disinfecting operation may take place while the harvesting apparatus 20 is performing a harvesting operation.

## Claims

1. A crop harvesting system comprising:
a moving apparatus (10) configured to be movable in a harvesting operation area (901), the harvesting operation area being an area in which a harvesting operation takes place;
a harvesting apparatus (20) mounted on the moving apparatus and automatically harvesting a harvesting object by making contact with a crop (T), the crop being a harvesting object;
a disinfecting apparatus (40) having a function of disinfecting at least a disinfection target part (221) of the harvesting apparatus, the disinfection target part being brought into contact with the harvesting object;
a disinfection necessity judgment processing portion (54) configured to perform a disinfection necessity judgment process of judging whether disinfection to the disinfection target part is necessary;
a disinfecting operation processing portion (55) configured to, when it is judged in the disinfection necessity judgment process that the disinfection is necessary, perform a disinfecting operation of disinfecting the disinfection target part by driving at least one of the harvesting apparatus and the disinfecting apparatus;
a location information obtaining apparatus (30) configured to obtain location information of a present location of the moving apparatus; and
a present location estimating processing apparatus (53) configured to perform a present location estimating process of estimating the present location of the moving apparatus with reference to the location information obtained by the location information obtaining apparatus, wherein
the disinfection necessity judgment process includes a process of judging that the disinfecting operation by the disinfecting apparatus is necessary when it is judged that the harvesting apparatus is going to start the harvesting operation, with reference to the present location of the moving apparatus, the present location being estimated in the present location estimating process, and
the disinfecting operation processing portion performs the disinfecting operation before the harvesting apparatus performs the harvesting operation.

2. The crop harvesting system according to claim 1, wherein
the disinfection necessity judgment process includes a process of judging that the disinfecting operation by the disinfecting apparatus is necessary when the moving apparatus is about to enter the harvesting operation area.

3. The crop harvesting system according to claim 1 or 2, wherein
the disinfection necessity judgment process includes a process of judging that the disinfecting operation by the disinfecting apparatus is necessary when the moving apparatus leaves the harvesting operation area.

4. The crop harvesting system according to any one of claims 1 to 3, wherein
the disinfecting operation processing portion makes the disinfecting apparatus perform the disinfecting operation while the present location of the moving apparatus is outside the harvesting operation area, the present location being estimated in the present location estimating process.

5. The crop harvesting system according to any one of claims 1 to 4, wherein
the location information obtaining apparatus obtains the present location of the moving apparatus by a method including at least one of an RFID, a two-dimensional code, a magnetic marker, a visual apparatus, and a GPS.

6. The crop harvesting system according to any one of claims 1 to 5, further comprising:
a map creation processing portion (51) configured to perform a map creating process of creating a map of the harvesting operation area in accordance with input by a worker or in accordance with visual information obtained from a visual apparatus (30), wherein
the present location estimating process includes a process of estimating the present location of the moving apparatus with reference to the map of the harvesting operation area, the map being created in the map creating process.

7. The crop harvesting system according to any one of claims 1 to 6, further comprising:
a traveled quantity obtainment processing portion (52) configured to perform a traveled quantity obtaining process of obtaining a quantity traveled by the moving apparatus, wherein
the present location estimating process further includes a process of estimating the present location of the moving apparatus with reference to the quantity traveled by the moving apparatus from a reference position.

8. The crop harvesting system according to any one of claims 1 to 7, wherein
the disinfecting apparatus includes a spraying portion (42) to eject disinfectant solution and performs the disinfection by spraying the disinfectant solution to the disinfection target part from the spraying portion.

9. The crop harvesting system according to claim 8, wherein
the disinfecting apparatus further includes an optical spraying sensor (46) configured to detect spraying of the disinfectant solution.

10. The crop harvesting system according to claim 8 or 9, wherein
the disinfecting apparatus further includes:
an opening portion (431) allowing the disinfection target part to be inserted through; and
a covering member (43) having at least one lateral face facing the direction in which the disinfectant solution is sprayed from the spraying portion.

11. The crop harvesting system according to any one of claims 8 to 10, wherein
the disinfecting apparatus further includes:
a disinfectant solution tank (41) to store the disinfectant solution;
a pump (44) to suck the disinfectant solution from the disinfectant solution tank 41; and
a three-way solenoid valve (45) to selectively switching between a spraying route (J) and a return route (R), the spraying route J connecting the pump and the spraying portion to feed to the spraying portion the disinfectant solution sprayed from the pump, the return route R connecting the pump and the disinfectant solution tank to return to the disinfectant solution tank the disinfectant solution sprayed from the pump, wherein
to perform the disinfecting operation, the disinfecting apparatus circulates the disinfectant solution by way of the disinfectant solution tank and the pump, by driving the pump while the three-way solenoid valve is set to the return route, and then ejects the disinfectant solution from the spraying portion by switching the three-way solenoid valve to the spraying route.

12. The crop harvesting system according to any one of claims 1 to 11, wherein
the disinfecting apparatus is mounted on the moving apparatus and configured to be movable along with the moving apparatus.

13. The crop harvesting system according to any one of claims 1 to 12, wherein
the harvesting apparatus makes the disinfection target part operate as in harvesting while the disinfecting apparatus is performing the disinfecting operation.

14. The crop harvesting system according to any one of claims 1 to 13, wherein
the harvesting apparatus is provided with:
an articulated robot arm (21); and
a harvesting tool (22) attached to an arm end part of the robot arm and to harvest the harvesting object in contact with the harvesting object, wherein
the harvesting apparatus performs an operation of changing a posture of the harvesting tool, by driving the robot arm while the disinfecting apparatus is performing the disinfecting operation.

## Patentansprüche

1. Feldfruchterntesystem, aufweisend:
eine Bewegungsvorrichtung (10), die dafür ausgelegt ist, in einem Erntevorgangsgebiet (901) bewegbar zu sein, wobei das Erntevorgangsgebiet ein Gebiet ist, in dem ein Erntevorgang stattfindet;
eine Erntevorrichtung (20), die an der Bewegungsvorrichtung befestigt ist und automatisch ein Ernteobjekt erntet, indem sie eine Feldfrucht (T) berührt, wobei die Feldfrucht ein Ernteobjekt ist;
eine Desinfektionsvorrichtung (40) mit einer Funktion des Desinfizierens zumindest eines Desinfektionszielteils (221) der Erntevorrichtung, wobei das Desinfektionszielteil mit dem Ernteobjekt in Kontakt gebracht wird;
einen Desinfektionsnotwendigkeitsbeurteilungsverarbeitungsabschnitt (54), der dafür ausgelegt ist, einen Desinfektionsnotwendigkeitsbeurteilungsvorgang durchzuführen, bei dem geurteilt wird, ob eine Desinfektion des Desinfektionszielteils notwendig ist;
einen Desinfektionsvorgangsverarbeitungsabschnitt (55), der dafür ausgelegt ist, wenn bei dem Desinfektionsnotwendigkeitsbeurteilungsvorgang geurteilt wird, dass die Desinfektion notwendig ist, einen Desinfektionsvorgang des Desinfizierens des Desinfektionszielteils durchführt, indem zumindest eines von der Erntevorrichtung und der Desinfektionsvorrichtung angetrieben wird;
eine Ortsinformationserhaltungsvorrichtung (30), die dafür ausgelegt ist, Ortsinformationen eines gegenwärtigen Orts der Bewegungsvorrichtung zu erhalten; und
eine Schätzverarbeitungsvorrichtung (53) für den gegenwärtigen Ort, die dafür ausgelegt ist, einen Schätzvorgang für den gegenwärtigen Ort durchzuführen, bei dem der gegenwärtige Ort der Bewegungsvorrichtung in Bezug auf die durch die Ortsinformationserhaltungsvorrichtung erhaltenen Ortsinformationen geschätzt wird, wobei
der Desinfektionsnotwendigkeitsbeurteilungsvorgang einen Vorgang des Urteilens beinhaltet, dass der Desinfektionsvorgang durch die Desinfektionsvorrichtung notwendig ist, wenn in Bezug auf den gegenwärtigen Ort der Bewegungsvorrichtung geurteilt wird, dass die Erntevorrichtung den Erntevorgang beginnen wird, wobei der gegenwärtige Ort während des Schätzvorgangs für den gegenwärtigen Ort geschätzt wird, und
wobei der Desinfektionsvorgangsverarbeitungsabschnitt den Desinfektionsvorgang durchführt, bevor die Erntevorrichtung den Erntevorgang durchführt.

2. Feldfruchterntesystem nach Anspruch 1, wobei
der Desinfektionsnotwendigkeitsbeurteilungsvorgang einen Vorgang des Urteilens beinhaltet, dass der Desinfektionsvorgang durch die Desinfektionsvorrichtung notwendig ist, wenn die Bewegungsvorrichtung dabei ist, in das Erntevorgangsgebiet einzutreten.

3. Feldfruchterntesystem nach Anspruch 1 oder 2, wobei
der Desinfektionsnotwendigkeitsbeurteilungsvorgang einen Vorgang des Urteilens beinhaltet, dass der Desinfektionsvorgang durch die Desinfektionsvorrichtung notwendig ist, wenn die Bewegungsvorrichtung das Erntevorgangsgebiet verlässt.

4. Feldfruchterntesystem nach einem der Ansprüche 1 bis 3, wobei
der Desinfektionsvorgangsverarbeitungsabschnitt die Desinfektionsvorrichtung veranlasst, den Desinfektionsvorgang durchzuführen, während sich der gegenwärtige Ort der Bewegungsvorrichtung außerhalb des Erntevorgangsgebiets befindet, wobei der gegenwärtige Ort während des Schätzvorgangs für den gegenwärtigen Ort geschätzt wird.

5. Feldfruchterntesystem nach einem der Ansprüche 1 bis 4, wobei
die Ortsinformationserhaltungsvorrichtung den gegenwärtigen Ort der Bewegungsvorrichtung durch ein Verfahren erhält, das zumindest eines von einer RFID, einem zweidimensionalen Code, einer Magnetmarkierung, einer visuellen Vorrichtung und einem GPS beinhaltet.

6. Feldfruchterntesystem nach einem der Ansprüche 1 bis 5, ferner aufweisend:
einen Kartenerzeugungsverarbeitungsabschnitt (51), der dafür ausgelegt ist, einen Kartenerzeugungsvorgang durchzuführen, bei dem eine Karte des Erntevorgangsgebiets in Übereinstimmung mit einer Eingabe eines Arbeiters oder in Übereinstimmung mit visuellen Informationen, die von einer visuellen Vorrichtung (30) erhalten werden, erzeugt wird, wobei
der Schätzvorgang für den gegenwärtigen Ort einen Vorgang des Schätzens des gegenwärtigen Orts der Bewegungsvorrichtung in Bezug auf die Karte des Erntevorgangsgebiets beinhaltet, wobei die Karte bei dem Kartenerzeugungsvorgang erzeugt wird.

7. Feldfruchterntesystem nach einem der Ansprüche 1 bis 6, ferner aufweisend:
einen Fortbewegungsstrecke-Erhaltungsverarbeitungsabschnitt (52), der dafür ausgelegt ist, einen Fortbewegungsstrecke-Erhaltungsvorgang durchzuführen, bei dem eine Fortbewegungsstrecke erhalten wird, die die Bewegungsvorrichtung zurückgelegt hat, wobei
der Schätzvorgang für den gegenwärtigen Ort ferner einen Vorgang des Schätzens des gegenwärtigen Orts der Bewegungsvorrichtung in Bezug auf die Fortbewegungsstrecke, die die Bewegungsvorrichtung ab einer Referenzposition zurückgelegt hat, beinhaltet.

8. Feldfruchterntesystem nach einem der Ansprüche 1 bis 7, wobei
die Desinfektionsvorrichtung einen Sprühabschnitt (42) beinhaltet, um Desinfektionslösung auszustoßen, und die Desinfektion dadurch durchführt, dass sie die Desinfektionslösung von dem Sprühabschnitt auf das Desinfektionszielteil sprüht.

9. Feldfruchterntesystem nach Anspruch 8, wobei
die Desinfektionsvorrichtung ferner einen optischen Sprühsensor (46) beinhaltet, der dafür ausgelegt ist, das Sprühen der Desinfektionslösung zu erfassen.

10. Feldfruchterntesystem nach Anspruch 8 oder 9, wobei
die Desinfektionsvorrichtung ferner beinhaltet:
einen Öffnungsabschnitt (431), durch den das Desinfektionszielteil eingeführt werden kann; und
ein Abdeckelement (43) mit zumindest einer lateralen Fläche, die in die Richtung gerichtet ist, in die die Desinfektionslösung von dem Sprühabschnitt gesprüht wird.

11. Feldfruchterntesystem nach einem der Ansprüche 8 bis 10, wobei
die Desinfektionsvorrichtung ferner beinhaltet:
einen Desinfektionslösungstank (41), um die Desinfektionslösung darin aufzunehmen;
eine Pumpe (44), um die Desinfektionslösung aus dem Desinfektionslösungstank (41) zu saugen; und
ein Dreiweg-Magnetventil (45), um selektiv zwischen einem Sprühpfad (J) und einem Rückführpfad (R) umzuschalten, wobei der Sprühpfad J die Pumpe und den Sprühabschnitt verbindet, um dem Sprühabschnitt die von der Pumpe gesprühte Desinfektionslösung zuzuführen, wobei der Rückführpfad R die Pumpe und den Desinfektionslösungstank verbindet, um die von der Pumpe gesprühte Desinfektionslösung zu dem Desinfektionslösungstank zurückzuführen, wobei
um den Desinfektionsvorgang durchzuführen, die Desinfektionsvorrichtung die Desinfektionslösung über den Desinfektionslösungstank und die Pumpe zirkuliert, indem die Pumpe angetrieben wird, während das Dreiweg-Magnetventil auf den Rückführpfad eingestellt ist, und dann die Desinfektionslösung aus dem Sprühabschnitt ausstößt, indem das Dreiweg-Magnetventil auf den Sprühpfad umgeschaltet wird.

12. Feldfruchterntesystem nach einem der Ansprüche 1 bis 11, wobei
die Desinfektionsvorrichtung an der Bewegungsvorrichtung befestigt und so konfiguriert ist, dass sie zusammen mit der Bewegungsvorrichtung bewegbar ist.

13. Feldfruchterntesystem nach einem der Ansprüche 1 bis 12, wobei
die Erntevorrichtung das Desinfektionszielteil veranlasst, sich wie bei einer Ernte zu verhalten, während die Desinfektionsvorrichtung den Desinfektionsvorgang durchführt.

14. Feldfruchterntesystem nach einem der Ansprüche 1 bis 13, wobei
die Erntevorrichtung mit Folgendem ausgestattet ist:
einem Roboter-Knickarm (21); und
einem Erntewerkzeug (22), das an dem Armendteil des Roboterarms befestigt ist und dazu dient, das Ernteobjekt durch Berührung mit dem Ernteobjekt zu ernten, wobei
die Erntevorrichtung einen Vorgang des Änderns einer Stellung des Erntewerkzeugs durchführt, indem der Roboterarm angetrieben wird, während die Desinfektionsvorrichtung den Desinfektionsvorgang durchführt.

## Revendications

1. Système de récolte de cultures, comprenant
un appareil mobile (10) configuré pour être déplacé dans une zone d'opération de récolte (901), la zone d'opération de récolte étant une zone dans laquelle une opération de récolte a lieu ;
un appareil de récolte (20) monté sur l'appareil mobile et récoltant automatiquement un objet à récolter en entrant en contact avec une culture (T), la culture étant un objet à récolter ;
un appareil de désinfection (40) ayant pour fonction de désinfecter au moins une partie cible de désinfection (221) de l'appareil de récolte, la partie cible de désinfection étant mise en contact avec l'objet à récolter ;
une partie de traitement de jugement de la nécessité de désinfection (54) configurée pour exécuter un processus de jugement de la nécessité de désinfection consistant à juger si la désinfection de la partie cible de désinfection est nécessaire ;
une partie de traitement de l'opération de désinfection (55) configurée pour, lorsqu'il est jugé dans le processus de jugement de la nécessité de désinfection que la désinfection est nécessaire, effectuer une opération de désinfection de la partie cible de désinfection en entraînant au moins l'un parmi l'appareil de récolte et l'appareil de désinfection ;
un appareil d'obtention d'informations de l'emplacement (30) configuré pour obtenir des informations d'un emplacement actuel de l'appareil mobile ; et un appareil de traitement d'estimation de l'emplacement actuel (53) configuré pour exécuter un processus d'estimation de l'emplacement actuel consistant à estimer l'emplacement actuel de l'appareil mobile par rapport aux informations d'emplacement obtenues par l'appareil d'obtention d'informations de l'emplacement,
dans lequel
le processus de jugement de la nécessité de désinfection comprend un processus consistant à juger que l'opération de désinfection par l'appareil de désinfection est nécessaire lorsqu'il est jugé que l'appareil de récolte est en train de commencer l'opération de récolte, par rapport à l'emplacement actuel de l'appareil mobile, l'emplacement actuel étant estimé dans le processus d'estimation de l'emplacement actuel, et
la partie de traitement de l'opération de désinfection effectue l'opération de désinfection avant que l'appareil de récolte n'effectue l'opération de récolte.

2. Système de récolte de cultures selon la revendication 1, dans lequel
le processus de jugement de la nécessité de désinfection comprend un processus consistant à juger que l'opération de désinfection par l'appareil de désinfection est nécessaire lorsque l'appareil mobile est sur le point d'entrer dans la zone d'opération de récolte.

3. Système de récolte de cultures selon la revendication 1 ou 2, dans lequel
le processus de jugement de la nécessité de désinfection comprend un processus consistant à juger que l'opération de désinfection par l'appareil de désinfection est nécessaire lorsque l'appareil mobile quitte la zone d'opération de récolte.

4. Système de récolte de cultures selon l'une des revendications 1 à 3, dans lequel
la partie de traitement de l'opération de désinfection assure que l'appareil de désinfection effectue l'opération de désinfection alors que l'emplacement actuel de l'appareil mobile se trouve en dehors de la zone d'opération de récolte, l'emplacement actuel étant estimé dans le processus d'estimation de l'emplacement actuel.

5. Système de récolte de cultures selon l'une des revendications 1 à 4, dans lequel
l'appareil d'obtention d'informations de l'emplacement obtient l'emplacement actuel de l'appareil mobile par une méthode comprenant au moins l'un des éléments suivants : RFID, code bidimensionnel, marqueur magnétique, appareil visuel et GPS.

6. Système de récolte de cultures selon l'une des revendications 1 à 5, comprenant en outre
une partie de traitement de création de carte (51) configurée pour exécuter un processus de création de carte de la zone d'opération de récolte en fonction des entrées d'un travailleur ou en fonction des informations visuelles obtenues à partir d'un appareil visuel (30),
dans lequel
le processus d'estimation de l'emplacement actuel comprend un processus d'estimation de l'emplacement actuel de l'appareil mobile par rapport à la carte de la zone d'opération de récolte, la carte étant créée au cours du processus de création de carte.

7. Système de récolte de cultures selon l'une des revendications 1 à 6, comprenant en outre :
une partie de traitement d'obtention de la quantité parcourue (52) configurée pour exécuter un processus d'obtention de la quantité parcourue consistant à obtenir une quantité parcourue par l'appareil mobile,
dans lequel
le processus d'estimation de l'emplacement actuel comprend en outre un processus d'estimation de l'emplacement actuel de l'appareil mobile par rapport à la quantité parcourue par l'appareil mobile à partir d'une position de référence.

8. Système de récolte de cultures selon l'une des revendications 1 à 7, dans lequel
l'appareil de désinfection comprend une partie de pulvérisation (42) pour éjecter une solution désinfectante et effectue la désinfection en pulvérisant la solution désinfectante sur la partie cible de désinfection à partir de la partie de pulvérisation.

9. Système de récolte de cultures selon la revendication 8, dans lequel l'appareil de désinfection comprend en outre un capteur optique de pulvérisation (46) configuré pour détecter la pulvérisation de la solution désinfectante.

10. Système de récolte de cultures selon la revendication 8 ou 9, dans lequel l'appareil de désinfection comprend en outre :
une partie d'ouverture (431) permettant d'insérer la partie cible de désinfection ; et
un élément de recouvrement (43) ayant au moins une face latérale orientée vers la direction dans laquelle la solution désinfectante est pulvérisée à partir de la partie de pulvérisation.

11. Système de récolte de cultures selon l'une des revendications 8 à 10, dans lequel
l'appareil de désinfection comprend en outre :
un réservoir de solution désinfectante (41) pour stocker la solution désinfectante ;
une pompe (44) pour aspirer la solution désinfectante du réservoir de solution désinfectante (41) ; et
une électrovanne à trois voies (45) pour commuter sélectivement entre une voie de pulvérisation (J) et une voie de retour (R), la voie de pulvérisation (J) reliant la pompe et la partie de pulvérisation pour alimenter la partie de pulvérisation en solution désinfectante pulvérisée par la pompe, la voie de retour (R) reliant la pompe et le réservoir de solution désinfectante pour renvoyer au réservoir de solution désinfectante la solution désinfectante pulvérisée par la pompe,
dans lequel
pour effectuer l'opération de désinfection, l'appareil de désinfection fait circuler la solution désinfectante via le réservoir de solution désinfectante et la pompe, en entraînant la pompe alors que l'électrovanne à trois voies est réglée sur la voie de retour, puis éjecte la solution désinfectante de la partie de pulvérisation en commutant l'électrovanne à trois voies sur la voie de pulvérisation.

12. Système de récolte de cultures selon l'une des revendications 1 à 11, dans lequel
l'appareil de désinfection est monté sur l'appareil mobile et est configuré pour être déplacé avec l'appareil mobile.

13. Système de récolte de cultures selon l'une des revendications 1 à 12, dans lequel
l'appareil de récolte fait fonctionner la partie cible de désinfection comme pour la récolte, pendant que l'appareil de désinfection effectue l'opération de désinfection.

14. Système de récolte de cultures selon l'une des revendications 1 à 13, dans lequel
l'appareil de récolte comprend :
un bras de robot (21) articulé ; et
un outil de récolte (22) fixé à une partie d'extrémité du bras de robot et destiné à récolter l'objet à récolter en contact avec l'objet à récolter, et
l'appareil de récolte effectue une opération de changement d'orientation de l'outil de récolte en entraînant le bras de robot pendant que l'appareil de désinfection effectue l'opération de désinfection.
